# EUROPEAN PATENT SPECIFICATION

(11) **EP 0 365 849 B1**
(45) Date of publication and mention of the grant of the patent: **14.09.1994**
(21) Application number: 89117735.4
(22) Date of filing: 26.09.1989
(51) Int. Cl.: C07H 19/16, A61K 31/70

(54) **Novel 2'-halomethylidene, 2'-ethenylidene and 2'-ethynyl adenosine derivatives**
2'-Halomethyliden, 2'-Ethenyliden und 2'-Ethynyl-Adenosinderivate
Dérivés 2'halométhylidène, 2'-éthenylidène et 2'-éthynyl d'adénosine

(30) Priority: 27.09.1988 US 249911; 03.08.1989 US 389391
(43) Date of publication of application: 02.05.1990
(73) Proprietor: MERRELL DOW PHARMACEUTICALS INC., Cincinnati Ohio 45215-6300 (US)
(72) Inventor: McCarthy, James R., West Chester Ohio 45069 (US); Stemerick, David M., Fairfield Ohio 45014 (US); Prakash, Nellikunja J., Cincinnati Ohio 45241 (US); Edwards, Michael L., Cincinnati Ohio 45240 (US); Jarvi, Esa T., Cincinnati Ohio 45236 (US)
(74) Representative: VOSSIUS & PARTNER

(56) References cited:
- EP-A- 0 304 889
- EP-A- 0 310 673
- CHEMICAL AND PHARMACEUTICAL BULLETIN OF JAPAN, vol. 34, no. 4, April 1986, pages 1518-1523, Pharmaceutical Society of Japan, Tokyo, JP; H. USUI et al.: "Synthesis of 8,2'-methano- and 8,2'-ethanoadenosines (Nucleosides and nucleotides. LXV)"

## Description

S-Adenosyl-L-methionine (AdoMet) dependent transmethylation reactions have been implicated in a variety of biological processes related to viral growth and replication, viral transformation of cells, growth of malignant cells, and processes such as chemotaxis and secretion [See P. M. Ueland, Pharm. Reviews, 34, 223 (1982)]. In general, these transmethylation reactions are catalyzed by various transmethylases which utilize AdoMet as a methyl-donor substrate in the methylation of a number of methyl-acceptor substrates such as catechols; norepinephrine; histamine; serotonin; tryptamine; membrane phospholipids; lysyl, arginyl, histidyl, aspartyl, glutamyl, and carboxyl groups of certain proteins; tRNA and mRNA; and DNA. These various transmethylases produce S-Adenosine-L-Homocysteine (AdoHcy) as a byproduct upon transfer of a methyl group from AdoMet to the appropriate methyl-acceptor substrate.

AdoHcy has been shown to be a potent feed-back inhibitor of the AdoMet-dependent transmethylation reactions. This feed-back inhibition of the transmethylases is controlled by the biodegradation of AdoHcy by S-Adenosyl-L-Homocysteine Hydrolase which provides a homeostatic control on the tissue levels of AdoHcy. The activity of S-Adenosyl-L-Homocysteine Hydrolase is generally considered by those skilled in the art to play an important role in regulating the tissue levels of AdoHcy and thereby controlling the activity of the AdoMet dependent transmethylation reactions.

The compounds of the present invention are inhibitors of S-Adenosyl-L-Homocysteine Hydrolase. These compounds therefore inhibit the naturally-occurring biodegradation of AdoHcy and result in elevated tissue levels of AdoHcy. Elevated levels of AdoHcy in turn provide an endogenous feed-back inhibition of various AdoMet dependent transmethylation reactions which are associated with biological processes related to viral growth and replication, viral transformation of cells, growth of malignant cells, and processes such as chemotaxis and secretion. The compounds of the present invention are therefore useful as inhibitors of these biological processes and useful in an end use application as therapeutic agents in the treatment of patients afflicted with various pathological conditions in which these processes are implicated, such as, viral infections and neoplastic disease states.

The present invention relates to novel 2'-halomethylidene, 2'-ethenylidene and 2'-ethynyl adenosine derivatives which are useful as inhibitors of S-Adenosyl-L-Homocysteine Hydrolase and are useful as antiviral and antineoplastic agents.

The present invention provides novel 2'-halomethylidene derivatives of the formula (1) 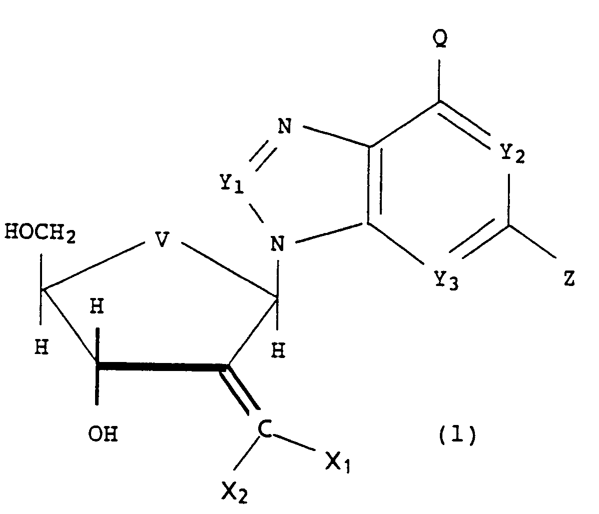
wherein
- V: is oxy, methylene, or thio,
- X₁ and X₂: are each independently hydrogen or halogen, with the proviso that at least one of X₁ and X₂ is halogen,
- Y₁: is nitrogen, a CH group, a CCl group, a CBr group or a CNH₂ group,
- Y₂ and Y₃: are each independently nitrogen or a CH group,
- Q: is NH₂, NHOH, NHCH₃, or hydrogen, and
- Z: is hydrogen, halogen, or NH₂;
or a pharmaceutically acceptable salt thereof.

In addition, the present invention also provides novel 2′-ethenylidene derivatives of the formula (1a) 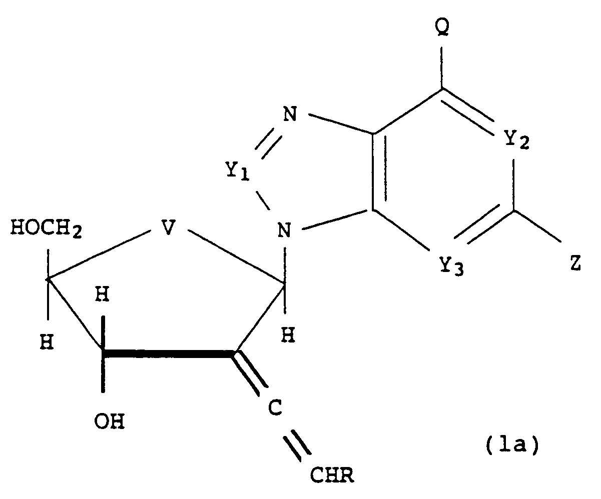
wherein
- V: is oxy, methylene, or thio,
- R: is hydrogen or C₁-C₄ alkyl,
- Y₁: is nitrogen, a CH group, a CCl group, a CBr group or a CNH₂ group,
- Y₂ and Y₃: are each independently nitrogen or a CH group,
- Q: is NH₂, NHOH, NHCH₃, or hydrogen, and
- Z: is hydrogen, halogen, or NH₂;
or a pharmaceutically acceptable salt thereof.

Furthermore, the present invention provides novel 2′-ethynyl derivatives of the formula (1b) 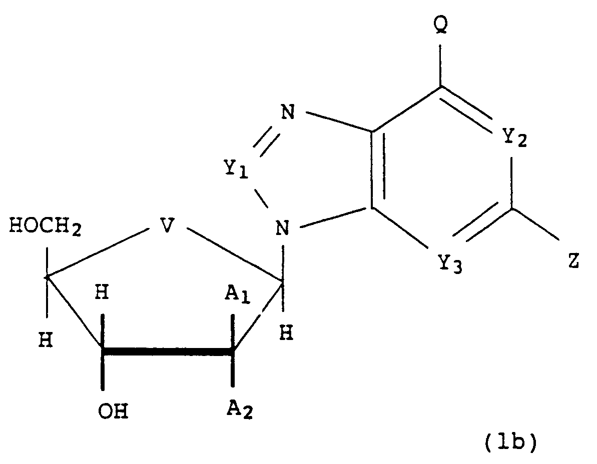
wherein
- V: is oxy, methylene, or thio,
- A₁ and A₂: are each independently hydrogen or a -C≡CR group,
wherein R is hydrogen or C₁-C₄ alkyl, with the proviso that where A₁ is hydrogen A₂ is a -C≡CR group, and where A₁ is a -C≡CR group A₂ is hydrogen,
- Y₁: is nitrogen, a CH group, a CCl group, a CBr group or a CNH₂ group,
- Y₂ and Y₃: are each independently nitrogen or a CH group,
- Q: is NH₂, NHOH, NHCH₃, or hydrogen, and
- Z: is hydrogen, halogen, or NH₂;
or a pharmaceutically acceptable salt thereof.

The present invention also provides a method of inhibiting AdoMet-dependent transmethylation activity in a patient in need thereof comprising administration of a therapeutically effective inhibitory amount of a compound of formula (1), (1a) or (1b).

Another embodiment of the present invention is a method of treating a patient afflicted with a neoplastic disease state or in controlling the growth of a neoplasm in a patient afflicted with a neoplastic disease state comprising administration of a therapeutically effective antineoplastic dose of a compound of formula (1), (1a) or (1b).

A further embodiment of the present invention is a method of treating a patient afflicted with a viral infection or of controlling a viral infection in a patient afflicted therewith comprising administration of a therapeutically effective antiviral amount of a compound of formula (1), (1a) or (1b).

As used herein, the term "halogen" refers to a fluorine, chlorine, bromine, or iodine atom and the term "nitrogen" refers to a trivalent nitrogen atomattached to two radicals. The term "C₁-C₄ alkyl" refers to a saturated straight or branched chain hydrocarbyl radical of one to four carbon atoms.

The 2′-halomethylidene, 2′-ethenylidene and 2′-ethynyl adenosine derivatives of formula (1), (1a) or (1b) can be prepared by utilizing procedures and techniques well known and appreciated by one of ordinary skill in the art.

A general synthetic procedure for the preparation of compounds of formula (1) wherein both X₁ and X₂ are halogen is set forth in Scheme A. In the following schemes all substituents, unless otherwise indicated, are as previously defined. In addition, the term "φ" refers to a phenyl group; the term "X_{HAL}" refers to a halogen atom; the term "LP=" indicates an ylide moiety [for example, a difluoromethylidene phosphonate ylide can have the formula (φ)₂P(O)=C(F)₂]. 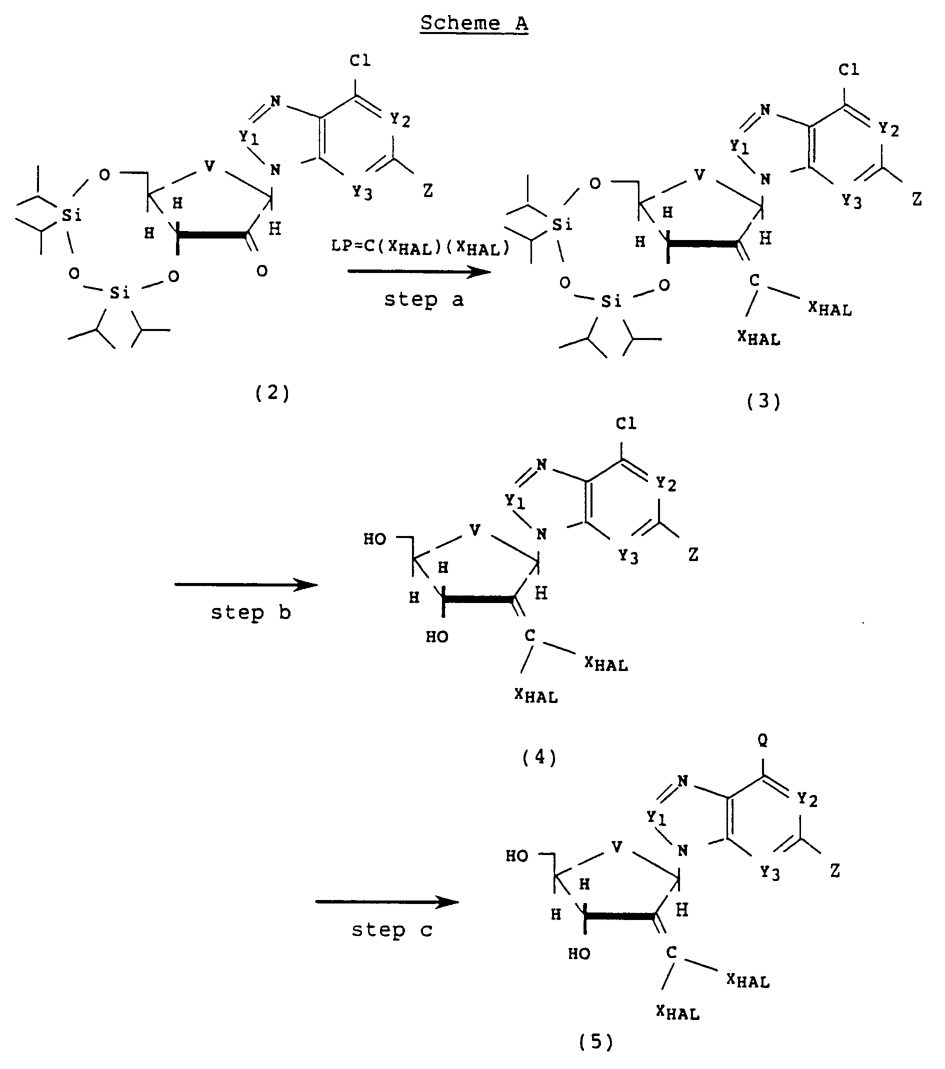
In step a, the ketone derivative (2), for example, 6-chloro-9-[( 3,5-O-tetraisopropyldisiloxan-1,3-diyl)-2-keto-β-D-erythro-pentofuranosyl]purine, can be reacted in a Wittig type reaction with a dihalomethylidene phosphorus ylide, to yield the corresponding 2-dihalomethylidene substituted derivative (3).

Phosphorus ylides can be prepared according to procedures which are well known and appreciated in the art of chemistry such as those described by J. March in "Advanced Organic Chemistry: Reactions, Mechanisms and Structure", McGraw-Hill Book Company, 702-10 (1968). For example, a phosphorus ylide can be prepared by treatment of an appropriate phosphorane or phosphonate derivative with an appropriate base. A wide variety of bases can be used including alkoxides and organometallics, such as alkyllithium or lithium dialkylamide. When a compound of formula (1) is desired wherein both X₁ and X₂ are halogen, a dihalomethylidene phosphorus ylide should be utilized in step a.

Appropriate phosphoranes or phosphonates can be prepared by addition of phosphines or phosphine oxides, such as trialkylphosphine, triarylphosphine (including triphenylphosphine) and diarylphosphine oxide (including diphenylphosphine oxide), to the appropriate di- or tri-halomethane derivative. The appropriate phosphorane or phosphonate is converted to the corresponding phosphorus ylide by treating the phosphorane or phosphonate with base. This can be accomplished by carrying out the preparation of the phosphorane or phosphonate in the presence of an appropriate base. When a compound of formula (1) is desired wherein both X₁ and X₂ are halogen, the appropriate ketone (2) can be reacted with a dihalomethylidene phosphorus ylide, prepared by reacting a phosphine or phosphine oxide with a trihalomethane in the presence of base.

More specifically, when a compound of formula (1) is desired wherein both X₁ and X₂ are fluorine, the appropriate ketone (2) is reacted with a difluoromethylidene phosphorus ylide, prepared by reacting a phosphine or phosphine oxide (such as diphenylphosphine oxide) with a difluorohalomethane (such as difluorochloromethane) in the presence of base (such as butyl lithium).

In step b, the tetraisopropyldisiloxan blocking group of (3) is removed, according to conventional procedures and techniques well known and appreciated in the art, to yield the de-blocked dihalomethylidene derivative (4). The tetraisopropyldisiloxan blocking group can be removed by reacting (3) with a fluoride anion or acid to effectively remove the blocking group without degradation of the desired product. For example, tetrabutylammonium fluoride or dilute hydrochloric acid can be used.

In step c, the 6-chloro moiety of the purine base of (4) is substituted with the desired moiety represented by the term "Q" to yield the 2′-dihalomethylidene adenosine derivative (5) of the present invention. This substitution can be effected according to procedures well known and appreciated in the art of chemistry. For example, where an NH₂ group is desired as Q, (4) can be reacted with methanolic ammonia to effect the substitution of an NH₂ group for the 6-chloro moiety.

The following example presents a typical synthesis as described by Scheme A. This example is understood to be illustrative only and is not intended to limit the scope of the present invention in any way.

### EXAMPLE 1

### 2′-DEOXY-2′-DIFLUOROMETHYLIDENE-ADENOSINE

### Step a: 6-Chloro-9-[(3,5-O-tetraisopropyldisiloxan-1,3-diyl)-β-D-erythro-pentofuran-2-(difluoromethylidene)osyl] purine

Prepare diphenyldifluoromethylphosphine oxide as follows: To a solution of diphenylphosphine oxide [25 grams (gm), 124 millimoles (mmol)] in tetrahydrofuran (THF) [600 milliliters (ml)] which has been cooled to -50 °Celsius (°C), add 70ml of a solution of 1.8 molar (M) n-butyl lithium in hexane and allow to stand at -50°C for 20 minutes (min). Add an excess of difluorochloromethane slowly and stir at -50°C for 3 hours. Allow the mixture to come to room temperature and evaporate the solvent *in vacuo*. Redissolve the residue in chloroform/water (1/1, v/v; 200ml). Separate the organic layer, dry with anhydrous magnesium sulfate, and evaporate to dryness. Purify by flash chromatography on silica gel eluting with toluene/ethyl acetate (1/1, v/v). Recrystallize from hexane/dichloromethane to yield the purified diphenyldifluoromethylphosphine oxide (melting point 93-94°C).

Cool diisopropylamine [1.7 ml, 12 mmol] in THF (24 ml) to - 20°C in a chloroform/dry ice bath. Add n-butyl lithium [8.88 ml of a 1.35 molar (M) solution in hexane] in a dropwise manner and stir the mixture for 20 min. Cool the mixture to -70°C in an acetone/dry ice bath. Add diphenyldifluoromethylphosphine oxide [3.02 gm, 12 mmol] in THF (12 ml) in a dropwise manner at such a rate that the temperature of the mixture does not rise above -65°C. Stir the mixture for 30 min and then add 6-chloro-9-[(3,5-O-tetraisopropyldisiloxan-1,3-diyl)-2-keto-β-D-erythro-pentofuranosyl]purine ( 5.26 gm, 10 mmol) in THF (20 ml) in a dropwise manner. Stir the mixture for 1 hour at -70°C, gradually warm the mixture to room temperature and then reflux for 1/2 hour. Cool the mixture to room temperature, add ethyl acetate (500 ml) and wash the organic mixture with saturated aqueous sodium bicarbonate (100 ml). Separate the organic layer, dry with anhydrous magnesium sulfate, and evaporate to dryness *in vacuo*. Chromatograph the residue on a silica gel flash column eluting with ethyl acetate/hexane (1/1, v/v) to yield the title compound.

### Step b: 6-chloro-9-[β-D-erythro-pentofuran-2-(difluoromethylidene)osyl]purine

To a solution of 1.0 M tetrabutylammonium fluoride in THF (2.2 ml, 2.2 mmol) add 6-chloro-9-[(3,5-O-tetraisopropyldisiloxan-1,3-diyl)-β-D-erythro-pentofuran-2-(difluoromethylidene)osyl]purine ( 560mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (9/1, v/v) to provide the title compound.

### Step c: 2′-Deoxy-2′-difluoromethylidene-adenosine

Heat a solution of 6-chloro-9-[β-D-erythro-pentofuran-2-(difluoromethylidene)osyl]purine ( 954 mg, 3 mmol) in methanolic ammonia (10 ml, saturated at 0°C) in a sealed tube at 100°C for 2 days. Evaporate the solution to dryness to provide the title compound.

The following compounds can be made by procedures analogous to those described above in Example 1:
2′-deoxy-2′-difluoromethylidene-N⁶-methyladenosine
2′-deoxy-2′-dichloromethylidene-adenosine
2′-deoxy-2′-difluoromethylidene-aristeromycin
2′-deoxy-2′-difluoromethylidene-4′-thio-adenosine
2′-deoxy-2′-difluoromethylidene-8-amino-adenosine
2′-deoxy-2′-difluoromethylidene-3-deaza-adenosine
2′-deoxy-2′-difluoromethylidene-1-deaza-adenosine
A general synthetic procedure for the preparation of compounds of formula (1) wherein one of X₁ and X₂ is hydrogen is set forth in Scheme B. 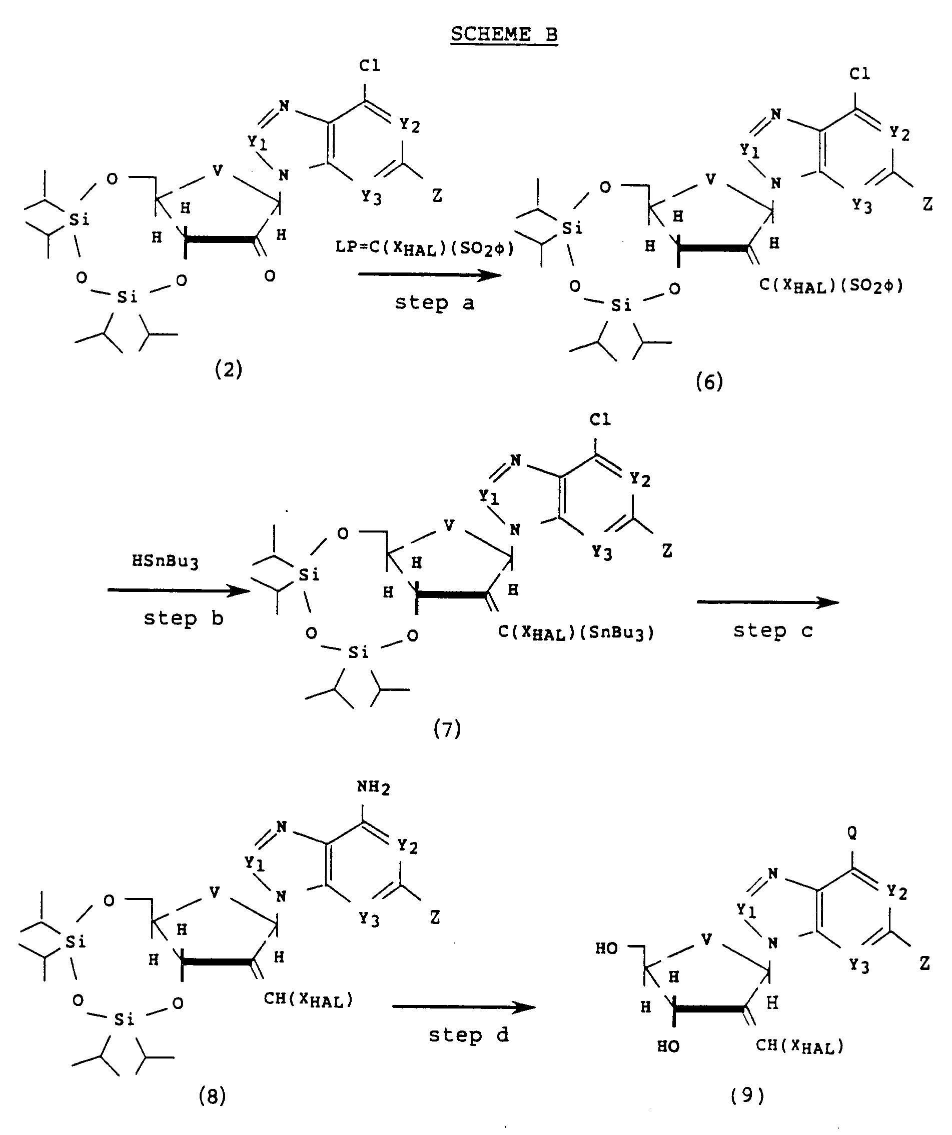
In step a, the ketone derivative (2), for example, 6-chloro-9-[(3,5-O-tetraisopropyldisiloxan-1,3-diyl)-2-keto-β-D-erythro-pentofuranosyl]purine, is reacted in a Wittig reaction with a phosphorus ylide as generally described for Scheme A. When a compound of formula (1) is desired wherein one of X₁ or X₂ is hydrogen, (2) can be reacted with a phenylsulfonyl-halomethylidene phosphorus ylide, to yield the corresponding 2-phenylsulfonyl-halomethylidene derivative (6).

The appropriate phenylsulfonyl-halomethylidene phosphorus ylide can be prepared according to procedures which are well known and appreciated in the art of chemistry. For example, where a compound of formula (1) is desired wherein one of X₁ or X₂ is hydrogen, the appropriate ketone (2) can be reacted with a phenylsulfonyl-halomethylidene phosphorus ylide prepared by reacting a halo phosphate (such as diethyl chlorophosphate) with a halomethylphenylsulfone in the presence of a base (such as lithium diisopropylamide).

More specifically, when a compound of formula (1) is desired wherein one of X₁ or X₂ is fluorine, the appropriate ketone (2) can be reacted with a phenylsulfonyl-fluoromethylidene phosphorus ylide prepared by reacting a halo phosphate (such as diethyl chlorophosphate) with fluoromethylphenylsulfone in the presence of a base (such as lithium diisopropylamide).

In step b, the 2-phenylsulfonyl-halomethylidene derivative (6) is converted to the corresponding 2-tributyl-tin-halomethylidene derivative (7). For example, this reaction can be accomplished by reacting (6) with tributyl-tin hydride (HSnBu₃) in the presence of 2,2′-azobis-isobutylnitrile (AIBN) in a suitable solvent such as benzene. The geometric isomers of the 2-tributyl-tin-halomethylidene derivative (7) can optionally be isolated using procedures and techniques which are well known and appreciated in the art. For example, the geometric isomers of (7) can be separated conveniently by Flash Chromatography (silica gel) eluting with 7% ethyl acetate in hexane.

In step c, the tributyl-tin moiety of (7) is removed and replaced by a hydrogen atom to provide the corresponding 2-halomethylidene derivative (8). This can be accomplished by reacting (7) with hexamethyldisilazane in the presence of a catalytic amount of ammonia in formamide. In addition, it will be recognized by those skilled in the art that in the course of removing the tributyl-tin moiety of (7), the 6-chloro moiety of the purine base of (7) will also be replaced with an amino moiety.

In step d, the tetraisopropyldisiloxan blocking group of (8) is removed as described for Scheme A (step b), to yield the corresponding de-blocked 2'-halomethylidene adenosine derivative (9). For example, a fluoride salt such as cesium fluoride can be used. In addition, where a compound of the present invention is desired wherein Q is other than amino, the 6-amino moiety of the purine base of (9) can be substituted with the desired moiety according to procedures well known and appreciated in the art of chemistry.

As is readily apparent to one skilled in the art, the 2'-halomethylidene adenosine derivative represented by the compound (9) in Scheme B, exists as two geometric isomers which can be referred to as the (Z) and the (E) isomers. These isomers can be separated using conventional separation techniques well known and appreciated in the art. For example, the geometric isomers may be resolved by column chromatography using a Dowex 1-X2 (OH⁻ form) resin. Optionally, the 2-tributyl-tin-halomethylidene derivative (7) or the corresponding 2-halomethylidene derivative (8) can conveniently be resolved into its geometric isomers using known techniques. The corresponding geometric isomers of the 2'-halomethylidene adenosine derivatives (9) will be formed by continuing the synthesis as outlined in Scheme B using the individual isomers of (7) or (8).

The following example presents a typical synthesis as described by Scheme B. This example is understood to be illustrative only and is not intended to limit the scope of the present invention in any way.

### EXAMPLE 2

### (Z)- and (E)-2'-DEOXY-2'-FLUOROMETHYLIDENE-ADENOSINE

### Step a: 6-Chloro-9-[(3,5-O-tetraisopropyldisiloxan-1,3-diyl)-β-D-erythro-pentofuran-2-(2-fluoro-2-phenylsulfonyl methylidene)osyl]purine

Prepare diethylfluoromethylphenylsulfonylphosphonate as follows: To a solution of fluoromethylphenyl sulfone (2.02 gm, 11.6 mmol) in dry THF (23 ml) which has been cooled to about -78°C in a dry 3-necked 100ml flask with stirring bar, argon inlet valve, thermometer and rubber septum, add diethyl chlorophosphate ( 4.02 gm, 3.37 ml, 11.6 mmol) via syringe. To this mixture, add a solution of 1.3 M lithium diisopropylamide in cyclohexane (17.7 ml, 23 mmol) via syringe and follow the formation of diethylfluoromethylphenylsulfonylphosphonate by gas chromatography (GC).

To the diethylfluoromethylphenylsulfonylphosphonate solution above which has been allowed to warm to 0°C, add a solution of 6-chloro-9-[(3,5-O-tetraisopropyldisiloxan-1,3-diyl)-2-keto-β-D-erythro-pentofuranosyl]purine (6.11 gm, 11.6 mmol) in dry THF (about 10 ml). Allow the reaction mixture to warm to room temperature and stir under an argon atmosphere for 4 hours. Pour the mixture into a saturated, ice-cold solution of ammonium chloride and extract the mixture with ethyl acetate (3 times, 75 ml each time). Combine the organic layers, dry with anhydrous magnesium sulfate, and evaporate to dryness. Chromatograph the residue on a silica gel flash column eluting with ethyl acetate/hexane (1/3, v/v). Evaporate the solvent to dryness under reduced pressure to provide the title compound as a white foam. Triturate with hexane, cool overnight, filter and dry the precipitate to provide 4.9 gm of the title compound.

### Step b: 6-Chloro-9-[(3,5-O-tetraisopropyldisiloxan-1,3-diyl)-β-D-erythro-pentofuran-2-(2-fluoro-2-tributyl-tin-methylidene)osyl]purine

Add tributyl-tin hydride (769 mg, 0.71 mL, 2.6 mmol) and 6-chloro-9-[(3,5-O-tetraisopropyldisiloxan-1,3-diyl)-β-D-erythropentofuran-2-(2-fluoro-2-phenylsulfonyl methylidene)osyl]purine (0.6 gm, 0.88 mmol) to benzene (8.8 mL) and heat to reflux. Add 2,2′-azobis-isobutylnitrile (35 mg, 0.26 mmol) in benzene (0.5 mL) over about 1 hour. Separate the isomers by flash chromatography (silica gel) eluting with 7% ethyl acetate in hexane to provide 0.31 gm of the first eluting isomer (fast-isomer) and 0.38 gm of the second eluting isomer (slow-isomer) of the title compound.

### Step c: (Z)- and (E)-6-Amino-9-[(3,5-O-tetraisopropyldisiloxan-1,3-diyl)-β-D-erythro-pentofuran-2-(2-fluoromethylidene)osyl]purine

Fast-isomer: Combine the fast isomer of 6-chloro-9-[(3,5-O-tetraisopropyldisiloxan-1,3-diyl)-β-D-erythro-pentofuran-2-(2-fluoro-2-tributyl-tin- methylidene)osyl]purine (0.7 gm, 0.8 mmol) and hexamethyldisilazane (3.5 mL) in formamide (1.75 mL) which has been saturated with ammonia. Heat the mixture to 100°C for 3 hours. Evaporate the solvent at reduced pressure and purify the residue by flash chromatography (silica gel) eluting with ethyl acetate/hexane (1/1, v/v). Evaporate the solvent to provide the title compound (0.165 gm, 0.31 mmol).

Slow-isomer: Combine the slow isomer of 6-chloro-9-[(3,5-O-tetraisopropyldisiloxan-1,3-diyl)-β-D-erythro-pentofuran-2-(2-fluoro-2-tributyl-tin- methylidene)osyl]purine (0.3 gm, 0.36 mmol) and hexamethyldisilazane (1.5 mL) in formamide (0.75 mL) which has been saturated with ammonia. Heat the mixture to 100°C for 3 hours. Evaporate the solvent at reduced pressure and purify the residue by flash chromatography (silica gel) eluting with ethyl acetate/hexane (1/1, v/v). Evaporate the solvent to provide the title compound (0.065 gm, 0.12 mmol).

### Step d: (Z)- and (E)-2′-Deoxy-2′-fluoromethylidene-adenosine

Fast-isomer: Combine 6-amino-9-[(3,5-O-tetraisopropyldisiloxan-1,3-diyl)-β-D-erythro-pentofuran-2-(2-fluoromethylidene)osyl]purine (fast isomer) (0.114 gm, 0.22 mmol) and cesium fluoride (1.32 gm, 8.7 mmol) in ethanol (30 mL) and stir at ambient temperature for about 24 hours. Evaporate the solvent in the presence of silica gel, place the silica gel residue on a flash chromatography column and elute with ethyl acetate followed by 10% ethanol in ethyl acetate. Evaporate the solvent to provide the title compound (47 mg, 0.16 mmol) in a 7.0% overall yield.

Slow-isomer: Combine 6-amino-9-[(3,5-O-tetraisopropyldisiloxan-1,3-diyl)-β-D-erythro-pentofuran-2-(2-fluoromethylidene)osyl]purine (slow isomer) (0.206 gm, 0.4 mmol) and cesium fluoride (2.28 gm, 15 mmol) in ethanol (60 mL) and stir at ambient temperature for about 24 hours. Evaporate the solvent in the presence of silica gel, place the silica gel residue on a flash chromatography column and elute with ethyl acetate followed by 10% methanol in ethyl acetate. Evaporate the solvent to provide the title compound (81 mg, 0.28 mmol) in a 5.9% overall yield.

The following compounds can be made by procedures analogous to those described above in Example 2:
(E) and (Z)-2′-deoxy-2′-fluoromethylidene-N⁶-methyladenosine
(E) and (Z)-2′-deoxy-2′-chloromethylidene-adenosine
(E) and (Z)-2′-deoxy-2′-fluoromethylidene-aristeromycin
(E) and (Z)-2′-deoxy-2′-fluoromethylidene-4′-thioadenosine
(E) and (Z)-2′-deoxy-2′-fluoromethylidene-8-aminoadenosine
(E) and (Z)-2′-deoxy-2′-fluoromethylidene-3-deazaadenosine
A general synthetic procedure for the preparation of compounds of formula (1a) wherein R₂ is hydrogen is set forth in Scheme C. 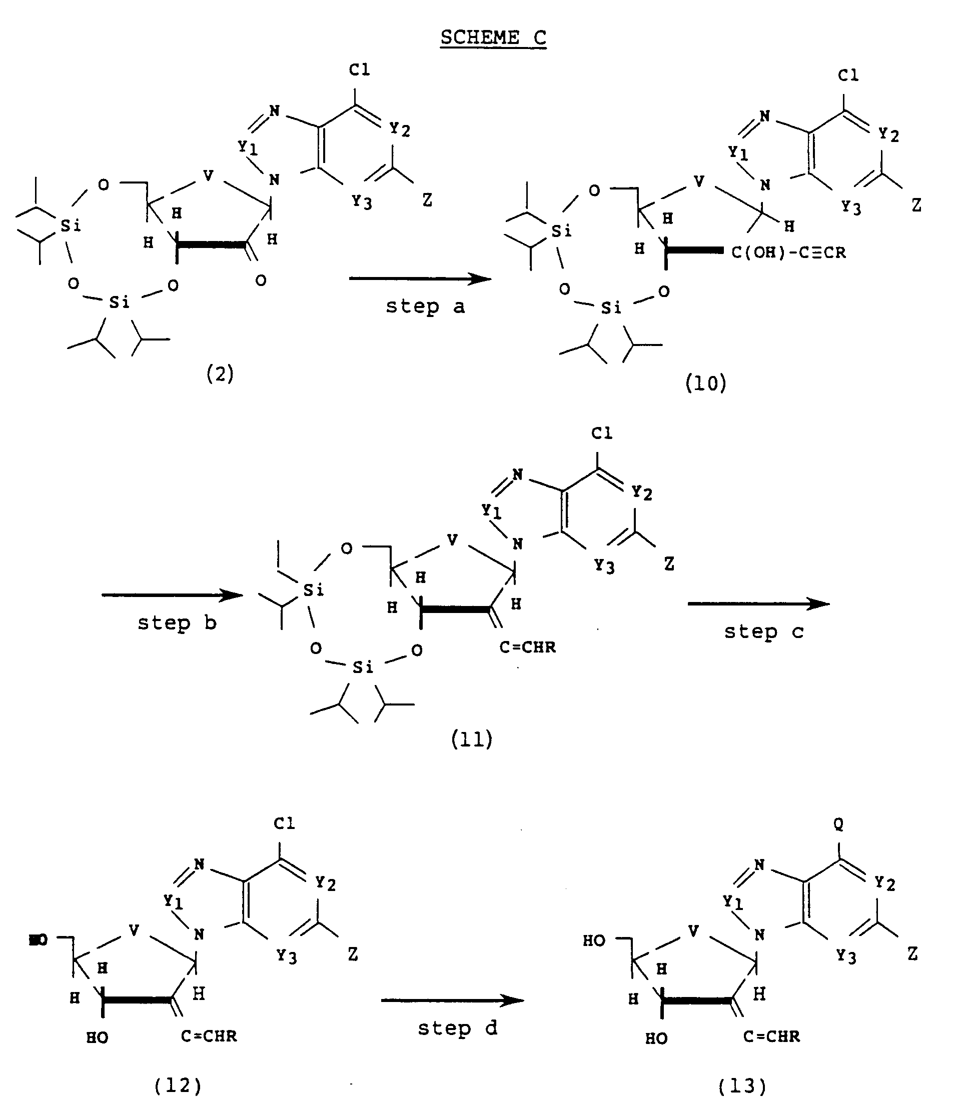
In step a, the ketone derivative (2), for example, 6-chloro-9-[(3,5-O-tetraisopropyldisiloxan-1,3-diyl)-2-keto-β-D-erythro-pentofuranosyl]purine, can be reacted with an acetylenic Grignard reagent such as that represented by the general formula R₁C≡CMgBr, to yield the corresponding 2-ethynyl alcohol (10). Alternatively, the alcohol (10) can be prepared from other organometallic compounds made from reactive metals, such as that represented by the general formula RC≡CLi.

The appropriate Grignard reagent, or other organometallic reagent, can be prepared according to methods and procedures which are well known and appreciated in the art of chemistry such as those described by J. March in "Advanced Organic Chemistry: Reactions, Mechanisms and Structure", McGraw-Hill Book Company, 684-88 and 697-98 (1968). For example, a Grignard reagent of acetylene or an alkyl-substituted acetylene can be prepared by treating acetylene or an alkyl-substituted acetylene with methyl magnesium bromide under anhydrous conditions.

It is of course well appreciated by one skilled in the art that the 2-ethynyl alcohol (10) can exist as one of two geometric isomers, i.e., one wherein the ethynyl group is on the same side of the furanosyl ring as the 3-hydroxy group and one wherein the ethynyl group is on the same side of the furanosyl ring as the purine group. These geometric isomers can be named 6-chloro-9-[(3,5-O-tetraisopropyldisiloxan-1,3-diyl)-β-D-ribo-pentofuran-2-(ethynyl)osyl]purine and 6-chloro-9-[(3,5-O-tetraisopropyldisiloxan-1,3-diyl)-β-D-arabinopentofuran-2-(ethynyl)osyl]purine, respectively.

In step b, the 2-ethynyl alcohol (10) is reduced to yield the 2-ethenylidene derivative (11). This reduction can be carried out according to methods and procedures which are well known and appreciated in the art of chemistry such as by treating the 2-ethynyl alcohol (10) with lithium aluminum hydride and aluminum chloride.

In step c, the tetraisopropyldisiloxan blocking group of (11) is removed as described for Scheme A (step b), to yield the corresponding de-blocked 2-ethenylidene derivative (12).

In step d, the 6-chloro moiety of the purine base of (12) is substituted with the desired moiety represented by the term "Q" to yield the 2′-ethenylidene adenosine derivative (13) of the present invention. This substitution can be effected according to procedures well known and appreciated in the art of chemistry as described for Scheme A (step d).

The following example presents a typical synthesis as described by Scheme C. This example is understood to be illustrative only and is not intended to limit the scope of the present invention in any way.

### EXAMPLE 3

### 2′-DEOXY-2′-ETHENYLIDENE-ADENOSINE

### Step a: 6-chloro-9-[(3,5-O-tetraisopropyldisiloxan-1,3-diyl)-β-D-(ribo or arabino)-pentofuran-2-(ethynyl)osyl]purine

At 0°C, saturate THF (750 ml) with acetylene and add 1.95 N methyl magnesium bromide (51 ml, 0.1 mol) in a dropwise manner while acetylene is still bubbling through the solution. Stop the acetylene stream 20 min after the addition of the methyl magnesium bromide is complete and purge for 20 min with argon. To this solution add 6-chloro-9-[(3,5-O-tetraisopropyldisiloxan-1,3-diyl)-2-keto-β-D-erythropentofuranosyl]purine (2.61 gm, 5 mmol) in THF (20 ml), warm the reaction mixture to room temperature and stir for 16 hours. Add 1600 ml of ethyl acetate and wash the mixture with saturated aqueous NH₄Cl (200 ml). Dry the organic layer with anhydrous magnesium sulfate and evaporate to dryness *in vacuo*. Chromatograph the residue on a silica gel flash column eluting with ethyl acetate/hexane (1/1, v/v) to provide the title compound.

### Step b: 6-chloro-9-[(3,5-O-tetraisopropyldisiloxan-1,3-diyl)-β-D-erythro-pentofuran-2-(ethenylidene)osyl] purine

To a stirred solution of lithium aluminum hydride (76 mg, 2 mmol) and aluminum chloride (132 mg, 1 mmol) in anhydrous diethyl ether (4 ml) which has been cooled to 0°C, add a solution of 6-chloro-9-[(3,5-O-tetraisopropyldisiloxan-1,3-diyl)-β-D-(ribo or arabino)-pentofuran-2-(ethynyl)osyl]purine (0.52 gm, 1 mmol) in anhydrous diethyl ether (2 ml) in a dropwise manner. Stir the reaction mixture for 1 hour and then quench the reaction by adding 10% potassium hydrogen sulfate (10 ml). Wash the aqueous solution with ethyl acetate (3 times, 20 ml each time). Combine the organic layers, dry with anhydrous magnesium sulfate, and concentrate the solution *in vacuo*. Chromatograph the residue on a silica gel flash column eluting with ethyl acetate/hexane (1/1, v/v) to provide the title compound.

### Step c: 6-Chloro-9-[β-D-erythro-pentofuran-2-(ethenylidene)osyl]purine

To a solution of 1.0 M tetrabutylammonium fluoride in THF (2.2 ml, 2.2 mmol) add 6-chloro-9-[(3,5-O-tetraisopropyldisiloxan-1,3-diyl)-β-D-erythro-pentofuran-2-(ethenylidene)osyl]purine (542 mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (20/1, v/v) to provide the title compound.

### Step d: 2′-Deoxy-2′-ethenylidene-adenosine

Heat a solution of 6-chloro-9-[β-D-erythro-pentofuran-2-(ethenylidene)osyl]purine (882 mg, 3 mmol) in methanolic ammonia (10 ml, saturated at 0°C) in a sealed tube at 100°C for 2 days. Evaporate the solution to dryness to provide the title compound.

The following compounds can be made by procedures analogous to those described above in Example 3:
2′-deoxy-2′-ethenylidene-N⁶-methyladenosine
2′-deoxy-2′-ethenylidene-aristeromycin
2′-deoxy-2′-ethenylidene-4′-thioadenosine
2′-deoxy-2′-ethenylidene-8-aminoadenosine
2′-deoxy-2′-ethenylidene-8-chloroadenosine
2′-deoxy-2′-ethenylidene-N⁶-hydroxyadenosine
2′-deoxy-2′-ethenylidene-3-deazaadenosine
A general synthetic procedure for the preparation of compounds of formula (1b) is set forth in Scheme D. 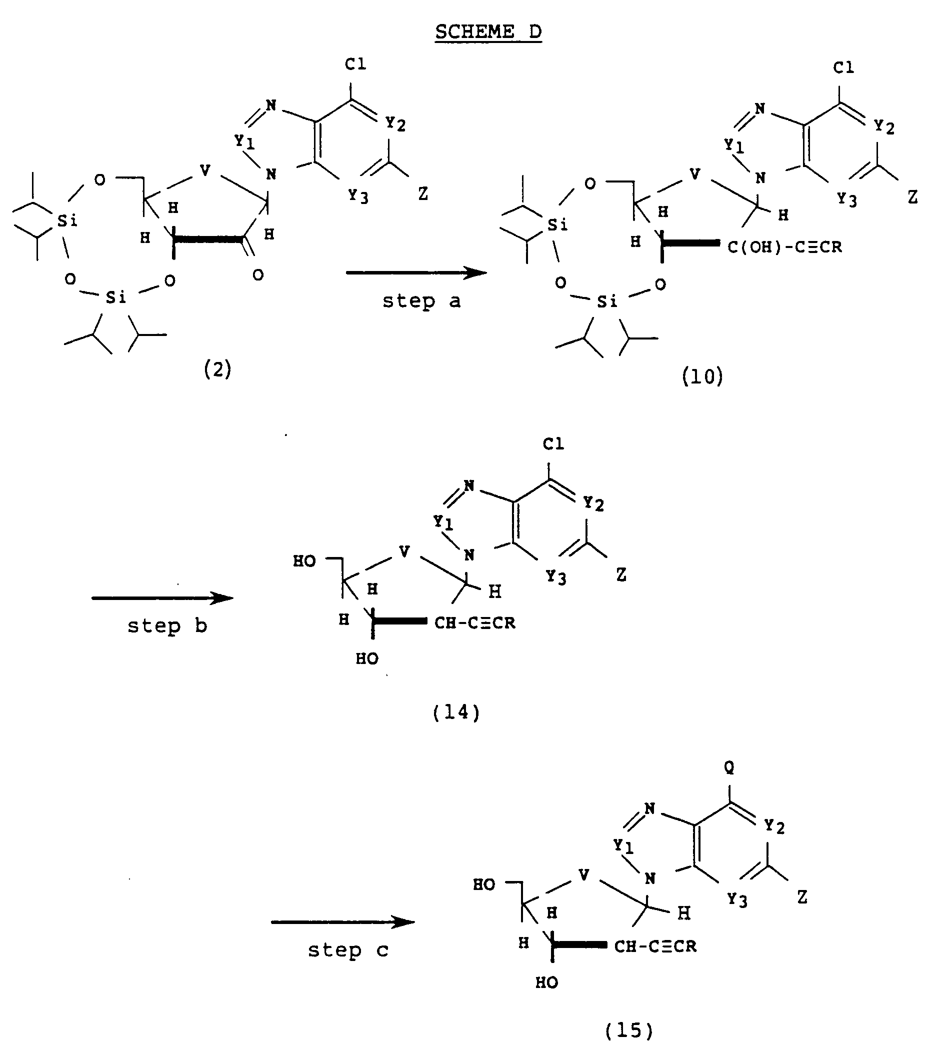
In step a, the ketone derivative (2), for example, 6-chloro-9-[(3,5-O-tetraisopropyldisiloxan-1,3-diyl)-2-keto-β-D-erythro-pentofuranosyl]purine, can be reacted as described in Scheme C (step a) to yield the corresponding 2-ethynyl alcohol (10). As described for Scheme C, the 2-ethynyl alcohol (10) can exist as two geometric isomers, i.e., 6-chloro-9-[(3,5-O-tetraisopropyldisiloxan-1,3-diyl)-β-D-ribo-pentofuran-2-(ethynyl)osyl]purine and 6-chloro-9-[(3,5-O-tetraisopropyldisiloxan-1,3-diyl)-β-D-arabino-pentofuran-2-(ethynyl)osyl] purine.

In step b, the 2-ethynyl alcohol (10) is reduced and the tetraisopropyldisiloxan blocking group is removed to yield the 2-ethynyl derivative (14). This reduction can be carried out according to methods and procedures which are well known and appreciated in the art of chemistry by treating the 2-ethynyl alcohol (10) with a reducing agent such as triethylsilane in the presence of an acid such as trifluoroacetic acid.

It is of course well appreciated by one skilled in the art that the 2-ethynyl derivative (14) can exist as one of two geometric isomers, i.e., one wherein the ethynyl group is on the same side of the furanosyl ring as the 3-hydroxy group and one wherein the ethynyl group is on the same side of the furanosyl ring as the purine group. These geometric isomers can be named 6-chloro-9-[β-D-erythro-pentofuran-2(R)-(ethynyl)osyl]purine and 6-chloro-9 [β-D-erythro-pentofuran-2(S)-(ethynyl)osyl]purine, respectively.

In step c, the 6-chloro moiety of the purine base of (14) is substituted with the desired moiety represented by the term "Q" to yield the 2′-ethynyl adenosine derivative (15) of the present invention. This substitution can be effected according to procedures well known and appreciated in the art of chemistry as described for Scheme A (step d). Again it is well appreciated by one skilled in the art that the 2′-ethynyl adenosine derivative (14) can exist as one of two geometric isomers, i.e., one wherein the ethynyl group is on the same side of the furanosyl ring as the 3-hydroxy group and one wherein the ethynyl group is on the same side of the furanosyl ring as the purine group. These geometric isomers can be named 2′-deoxy-2′(R)-ethynyl-adenosine (or 6-amino-9-[β-D-erythropentofuran-2(R)-(ethynyl)osyl]purine) and 2′-deoxy-2′(S)-ethynyl-adenosine (or 6-amino-9-[β-D-erythro-pentofuran-2(S)-(ethynyl)osyl]purine), respectively.

As is readily apparent to one skilled in the art, the (R) and the (S) geometric isomers of the 2′-ethynyl adenosine derivative (15) can be separated using conventional separation methods. For example, the geometric isomers may be resolved by column chromatography using methods and techniques which are well known and appreciated in the art.

The following example presents a typical synthesis as described by Scheme D. This example is understood to be illustrative only and is not intended to limit the scope of the present invention in any way.

### EXAMPLE 4

### 2′-DEOXY-2′(R or S)-ETHYNYL-ADENOSINE

### Step a: 6-Chloro-9-[(3,5-O-tetraisopropyldisiloxan-1,3-diyl)-β-D-(ribo or arabino)-pentofuran-2-(ethynyl)osyl]purine

Prepare the title compound as described in Example 3 (step a) above.

### Step b: 6-Chloro-9-[β-D-erythro-pentofuran-2(R and S)-(ethynyl)osyl]purine

Dissolve 6-chloro-9-[(3,5-O-tetraisopropyldisiloxan-1,3-diyl)-β-D-(ribo or arabino)-pentofuran-2-(ethynyl)osyl]purine (489 mg, 0.94 mmol) in dichloromethane (3ml) under a nitrogen atmosphere and cool the solution in an ice bath (0°C). Add trifluoroacetic acid (0.54 ml, 7.08 mmol) followed by triethylsilane (0.27 ml, 1.71 mmol) and stir the solution overnight at room temperature. Dilute the reaction mixture with ethyl acetate (10 ml) and wash with ice cold 1N sodium hydroxide solution (2 times, 5 ml each time). Dry the organic layer with anhydrous magnesium sulfate and evaporate to dryness.

### Step d: 2'-Deoxy-2'-ethynyl-adenosine and 9-(2-Ethynyl-β-D-arabinofuranosyl)adenine

Heat a solution of 6-chloro-9-[β-D-erythro-pentofuran-2(R or S)-(ethynyl)osyl]purine (880 mg, 3 mmol) in methanolic ammonia (10 ml, saturated at 0°C) in a sealed tube at 100°C for 2 days. Evaporate the solution to dryness and separate the arabino and ribo- compounds on a Dowex 1-X2 (OH⁻ form) chromatographic column to provide the title compounds.

The following compounds can be made by procedures analogous to those described above in Example 4:
2'-deoxy-2'(R or S)-ethynyl-N⁶-methyladenosine
2'-deoxy-2'(R or S)-ethynyl-aristeromycin
2'-deoxy-2'(R or S)-ethynyl-4'-thioadenosine
2'-deoxy-2'(R or S)-ethynyl-8-aminoadenosine
2'-deoxy-2'(R or S)-ethynyl-8-chloroadenosine
2'-deoxy-2'(R or S)-ethynyl-N⁶-hydroxyadenosine
2'-deoxy-2'(R or S)-ethynyl-3-deazaadenosine
Although in each of the reaction schemes presented for preparation of the compounds of the formula (1), (1a) and (1b), the starting material is exemplified as a 6-chloro-9-((3,5-O-tetraisopropyldisiloxan-1,3-diyl)-2-keto-β-D-erythro-pentofuranosyl]purine derivative, it is readily apparent to one skilled in the art that the compounds of the formula (1), (1a) and (1b) can be made by utilizing other 9-[(3,5-O-tetraisopropyldisiloxan-1,3-diyl)-2-keto-β-D-erythro-pentofuranosyl]-purine derivatives as the starting material in reaction schemes analogous to those presented.

Starting materials for use in the general synthetic procedure outlined in Schemes A through D are readily available by the use of synthetic methods and procedures which are well known and appreciated by those of ordinary skill in the art. For example, 6-amino-9-[(3,5-O-tetraisopropyldisiloxan-1,3-diyl)-2-keto-β-D-erythro-pentofuranosyl]purine can be used as the starting material for many of the compounds of formulas (1), (1a) and (1b) and can be prepared from adenosine according to the procedure described by Usui and Ueda [Chem. Pharm. Bull. 34, 15 (1986)]. 6-Chloro-9-[(3,5-O-tetraisopropyldisiloxan-1,3-diyl)-2-keto-β-D-erythro-pentofuranosyl]purine can be prepared from 6-chloro-9(β-D-ribofuranosyl)purine by a procedure analogous to that described by Usui and Ueda. Other 2-keto starting materials can be prepared by the use of methods analogous to those described by Usui and Ueda as well as other conventional methods as are well known and appreciated in the art.

In another embodiment, the present invention provides a method of inhibiting AdoMet-dependent transmethylation activity in a patient in need thereof which comprises administration of a compound of the formula (1), (1a) or (1b) in a therapeutically effective inhibitory amount. The term "therapeutically effective inhibitory amount" refers to an amount sufficient to inhibit the AdoMet-dependent transmethylation activity after single or multiple dose administration.

As used herein, the term "patient" refers to a warm-blooded animal such as a mammal which is afflicted with a particular disease state. It is understood that dogs, cats, rats, mice, horses, cattle, sheep, and humans are examples of animals within the scope of the meaning of the term.

The compounds of formula (1), (1a) or (1b) are believed to exert their inhibitory effect on AdoMet-dependent transmethylation by inhibition of AdoHcy Hydrolase thereby providing an increase in tissue levels of AdoHcy which in turn provides feedback inhibition of AdoMet-dependent transmethylation. However, it is understood that the present invention is not limited by any particular theory or proposed mechanism to explain its effectiveness in an end-use application.

As is well known and appreciated by those skilled in the art, various disease states, such as certain neoplastic disease states and viral infections, are characterized by excessive AdoMet-dependent transmethylation activity. As used herein, the term "excessive" means a level of activity which allows the disease state to progress.

More specifically, the present invention provides a method for the treatment of a patient afflicted with a neoplastic disease state which is characterized by excessive AdoMet dependent transmethylation activity comprising the administration thereto of a therapeutically effective antineoplastic amount of a compound of formula (1), (1a) or (1b). The term "neoplastic disease state" as used herein refers to an abnormal state or condition characterized by rapidly proliferating cell growth or neoplasm. Neoplastic disease states which are characterized by an excessive AdoMet-dependent transmethylation activity and for which treatment with a compound of formula (1), (1a) or (1b) will be particularly useful include: Leukemias such as, but not limited to, acute lymphoblastic, chronic lymphocytic, acute myoblastic and chronic myocytic; Carcinomas, such as, but not limited to, those of the cervix, oesophagus, stomach, small intestines, colon and lungs; Sarcomas, such as, but not limited to, oesteoma, osteosarcoma, lipoma, liposarcoma, hemangioma and hemangiosarcoma; Melanomas, including amelanotic and melanotic; and mixed types of neoplasias such as, but not limited to carcinosarcoma, lymphoid tissue type, folicullar reticulum cell sarcoma and Hodgkins Disease.

A therapeutically effective antineoplastic amount of a compound of formula (1), (1a) or (1b) refers to an amount which is effective, upon single or multiple dose administration to the patient, in controlling the growth of the neoplasm or in prolonging the survivability of the patient beyond that expected in the absence of such treatment. As used herein, "controlling the growth" of the neoplasm refers to slowing, interrupting, arresting or stopping its growth and metastases and does not necessarily indicate a total elimination of the neoplasm.

In addition, the present invention provides a method for the treatment of a patient afflicted with a viral infection which is characterized by excessive AdoMet-dependent transmethylation activity comprising the administration thereto of a therapeutically effective antiviral amount of a compound of formula (1), (1a) or (1b). The term "viral infection" as used herein refers to an abnormal state or condition characterized by viral transformation of cells, viral replication and proliferation. Viral infections which are characterized by an excessive AdoMet dependent transmethylation activity and for which treatment with a compound of formula (1), (1a) or (1b) will be particularly useful include: Retroviruses such as, but not limited to, HTLV-I, HTLV-II, human immunodeficiency viruses, HTLV-III (AIDS virus), and the like; RNA viruses such as, but not limited to, influenza type A, B, and C, mumps, measles, rhinovirus, dengue, rubella, rabies, hepatitis virus A, encephalitis virus, and the like; DNA viruses such as, but not limited to, herpes, vaccinia, papileoma virus (wart), hepatitis virus B, and the like.

A therapeutically effective antiviral amount of a compound of formula (1), (1a) or (1b) refers to an amount which is effective in controlling the virus. This viral control refers to slowing, interrupting, arresting or stopping the viral transformation of cells or the replication and proliferation of the virus and does not necessarily indicate a total elimination of the virus.

A therapeutically effective dose can be readily determined by the attending diagnostician, as one skilled in the art, by the use of conventional techniques and by observing results obtained under analogous circumstances. In determining the therapeutically effective dose, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances.

A therapeutically effective amount of a compound of formula (1), (1a) or (1b) is expected to vary from about 0.1 milligram per kilogram of body weight per day (mg/kg/day) to about 100 mg/kg/day. Preferred amounts are expected to vary from about 0.5 to about 10 mg/kg/day.

In an additional embodiment, the present invention relates to a method of treating a patient afflicted with a neoplastic disease state or a viral infection comprising administration thereto of a therapeutically effective antineoplastic or antiviral amount of a compound of formula (1), (1a) or (1b) wherein Q is NH₂ in conjunctive therapy with a therapeutically effective inhibitory amount of an Adenosine Deaminase (ADA) inhibitor. The term "conjunctive therapy" contemplates coadministration of (1), (1a) or (1b) along with an ADA inhibitor at essentially the same time, or treatment of the patient with an ADA inhibitor prior to or after treatment with a compound of formula (1), (1a) or (1b). A therapeutically effective inhibitory amount of an ADA inhibitor is an amount effective in significantly inhibiting ADA in the patient.

ADA deaminates compounds of formula (1), (1a) or (1b) wherein Q is NH₂ and thereby degrades the active compounds to relatively inactive metabolites. When a compound of formula (1), (1a) or (1b) wherein Q is NH₂ and an ADA inhibitor are administered in conjunctive therapy, the dose will be less in amount or frequency of administration than that required when the compound of formula (1), (1a) or (1b) is administered alone.

Various pharmaceutically acceptable non-toxic ADA inhibitors can be used including, but not limited to, deoxycoformycin. A therapeutically effective inhibitory amount of the ADA inhibitor will vary from about 0.05 mg/kg/day to about 0.5 mg/kg/day and preferably will be from about 0.1 mg/kg/day to about 0.3 mg/kg/day. Deoxycoformycin is the preferred ADA inhibitor for use in conjunctive therapy with compounds of formula (1), (1a) or (1b) wherein Q is NH₂.

In effecting treatment of a patient afflicted with a disease state described above, a compound of formula (1), (1a) or (1b) can be administered in any form or mode which makes the compound bioavailable in effective amounts, including oral and parenteral routes. For example, compounds of formula (1), (1a) or (1b) can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular characteristics of the compound selected the disease state to be treated, the stage of the disease, and other relevant circumstances.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. In addition, compounds of formula (1), (1a) or (1b) wherein Q is NH₂ can be administered as above in further combination with an ADA inhibitor. The compounds of the invention, while effective themselves, may be formulated and administered in the form of their pharmaceutically acceptable acid addition salts for purposes of stability, convenience of crystallization, increased solubility and the like.

In another embodiment, the present invention provides pharmaceutical compositions comprising a therapeutically effective amount of a compound of formula (1), (1a) or (1b) in admixture or otherwise in association with one or more pharmaceutically acceptable carriers or excipients. In addition, the present invention provides a pharmaceutical composition comprising a therapeutically effective amount of a compound of formula (1), (1a) or (1b) wherein Q is NH₂ and a therapeutically effective ADA inhibitory amount of an ADA inhibitor in admixture or otherwise in association with one or more pharmaceutically acceptable carriers or excipients. The term "therapeutically effective amounts" as applied to compounds of formula (1), (1a) or (1b) refers to effective inhibitory, antineoplastic, or antiviral amounts as appropriate.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the compounds of the present invention may be incorporated into a solution or suspension. These preparations should contain at least 0.1% of a compound of the invention, but may be varied to be between 0.1 and about 50% of the weight thereof. The amount of the inventive compound present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include the one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Any of the above described pharmaceutical compositions containing compounds of formula (1), (1a) or (1b) wherein Q is NH₂ may also contain a therapeutically effective inhibitory amount of an ADA inhibitor in admixture or otherwise in association with the above described ingredients.

As with any group of structurally related compounds which possesses a particular generic utility, certain groups and configurations are preferred for compounds of formula (1), (1a) or (1b) in their end-use application.

With respect to the substituents X₁ and X₂, compounds of formula (1) wherein X₁ is fluoro and X₂ is hydrogen, and those wherein X₁ is hydrogen and X₂ is fluoro, are generally preferred.

With respect to the substituent R, compounds of the formulas (1a) and (1b) wherein R is hydrogen are generally preferred.

The following are additional preferred embodiments for compounds of formula (1), (1a) or (1b): compounds wherein V is oxy, compounds wherein Y₁ is a CH group, compounds wherein Y₂ is nitrogen, compounds wherein Y₃ is nitrogen and compounds wherein Z is hydrogen are generally preferred. Finally, with respect to Q, those compounds of formula (1), (1a) or (1b) wherein Q is NH₂ or NHCH₃ are generally preferred with those wherein Q is NH₂ being especially preferred.

The following list identifies compounds of the formula (1),(1a) and (1b) which are particularly preferred embodiments of the present invention:
2′-deoxy-2′-difluoromethylidene-adenosine
2′-deoxy-2′-difluoromethylidene-aristeromycin
2′-deoxy-2′-difluoromethylidene-3-deazaadenosine
(Z) and (E) 2′-deoxy-2′-fluoromethylidene-adenosine
(Z) and (E) 2′-deoxy-2′-fluoromethylidene-aristeromycin
(Z) and (E) 2′-deoxy-2′-fluoromethylidene-3-deazaadenosine
2′-deoxy-2′-ethenylidene-adenosine
2′-deoxy-2′-ethenylidene-3-deazaadenosine
2′-deoxy-2′-ethenylidene-aristeromycin
2′-deoxy-2′(R) and (S)-ethynyl-adenosine
2′-deoxy-2′(R) and (S)-ethynyl-3-deazaadenosine
2′-deoxy-2′(R) and (S)-ethynyl-aristeromycin
The above list is intended to be merely illustrative of particularly preferred embodiments of the present invention and it is understood that the list does not limit the scope of the invention in any way.

## Claims (Claims for the following Contracting State(s): AT, BE, CH, DE, FR, GB, IT, LI, LU, NL, SE)

1. A compound of the general formula 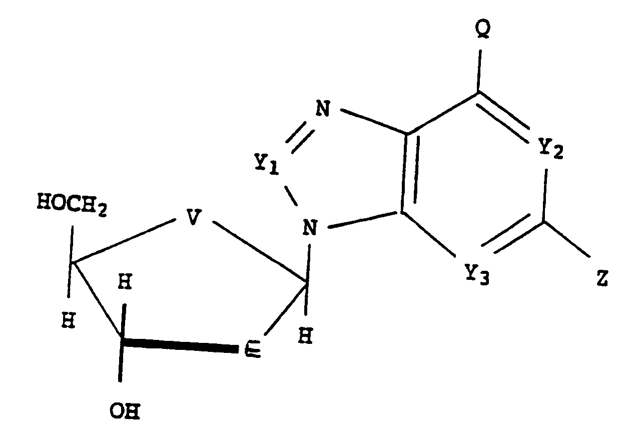 wherein
E is 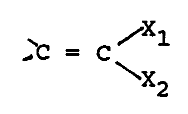 (X₁ and X₂ are each halogens, or one is hydrogen and the other halogen)
〉C = C = CHR (R is hydrogen or C₁-C₄-alkyl), or 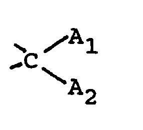 (one of A₁ and A₂ is hydrogen, the other -C≡C-R, wherein R is defined above),
V is oxy, methylene, or thio,
Y₁ is nitrogen, a CH group, a CCl group, a CBr group or a CNH₂ group,
Y₂ and Y₃ are each independently nitrogen or a CH group,
Q is NH₂, NHOH, NHCH₃, or hydrogen, and
Z is hydrogen, halogen, or NH₂;
or a pharmaceutically acceptable salt thereof.

2. A compound of claim 1 wherein E is 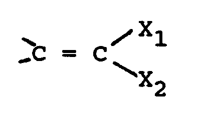 and X₁ is fluoro and X₂ is hydrogen.

3. A compound of claim 1 wherein E is 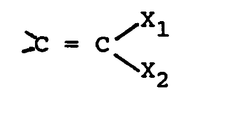 and X₁ is hydrogen and X₂ is fluoro.

4. A compound of claim 1 wherein E is
〉C = C = CHR or 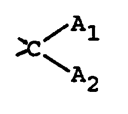 and R is hydrogen.

5. A compound of any one of claims 1 to 4 wherein V is oxy.

6. A compound of any one of claims 1 to 5 wherein Q is NH₂.

7. A compound of any one of Claims 1 to 6 wherein Z is hydrogen.

8. A compound of any one of claims 1 to 7 wherein Y₁ is a CH group.

9. A compound of any one of claims 1 to 8 wherein Y₂ is nitrogen.

10. A compound of any one of claims 1 to 8 wherein Y₂ is a CH group.

11. A compound of any one of claims 1 to 10 wherein Y₃ is nitrogen.

12. The compound of claim 1 wherein the compound is 2'-deoxy-2'-difluoromethylidene-adenosine.

13. The compound of claim 1 wherein the compound is (Z)-2'-deoxy-2'-fluoromethylidene-adenosine.

14. The compound of Claim 1 wherein the compound is (E)-2'-deoxy-2'-fluoromethylidene-adenosine.

15. The compound of claim 1 wherein the compound is 2'-deoxy-2'-ethenylidene-adenosine.

16. The compound of claim 1 wherein the compound is 2'-deoxy-2'(R)-ethynyl-adenosine.

17. The compound of claim 1 wherein the compound is 2'-deoxy-2'(S)-ethynyl-adenosine.

18. The compound of claim 1 wherein the compound is 2'-deoxy-2'-difluoromethylidene-aristeromycin.

19. The compound of claim 1 wherein the compound is 2'-deoxy-2'-difluoromethylidene-3-deaza-adenosine.

20. The compound of claim 1 wherein the compound is (E) or (Z)-2'-deoxy-2'-fluoromethylidene-aristeromycin.

21. The compound of claim 1 wherein the compound is (E) and (Z)-2'-deoxy-2'-fluoromethylidene-3-deazaadenosine.

22. The compound of claim 1 wherein the compound is 2'-deoxy-2'-ethenylidene-aristeromycin.

23. The compound of claim 1 wherein the compound is 2'-deoxy-2'-ethenylidene-3-deazaadenosine.

24. The compound of claim 1 wherein the compound is 2'-deoxy-2'(R or S)-ethynyl-aristeromycin.

25. The compound of claim 1 wherein the compound is 2'-deoxy-2'(R or S)-ethynyl-3-deazaadenosine.

26. A process for the preparation of a compound according to any one of claims 1, 5 to 12, 18 and 19, wherein E is 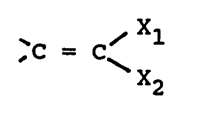 and X₁ and X₂ are each halogens
comprising the steps of
(a) reacting a 6-chloro-2'-keto-adenosine derivative, bearing appropriate blocking groups, with a dihalomethylidene phosphorus ylide to yield a 6-chloro-2'-dihalometylidene-adenosine derivative, bearing appropriate blocking groups,
(b) deblocking the 6-chloro-2'-dihalomethylidene-adenosine derivative by reacting with acid,
(c) substituting the 6-chloro moiety of the deblocked 6-chloro-2'-dihalomethylidene-adenosine derivative with a desired moiety as represented by the term "Q", and optionally
(d) converting the obtained compound to its pharmaceutically acceptable salt.

27. A process for the preparation of a compound according to any one of claims 1 to 3, 5 to 11, 13, 14, 20 and 21 wherein E is 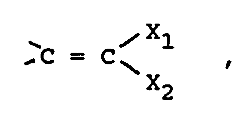 and one of X₁ and X₂ is hydrogen and the other halogen comprising the steps of
(a) reacting a 2'-phenylsulfonyl-halomethylidene adenosine derivative, bearing appropriate blocking groups, with tributyl-tin hydride in the presence of 2,2'-azobis-isobutylnitrile to form the corresponding 2'-tributyl-tin-halomethylidene derivative,
(b) reacting the 2'-tributyl-tin-halomethylidene derivative thus formed with hexamethyldisilasane in the presence of a catalytic amount of ammonia in formamide,
(c) removing the blocking groups, and optionally
(d) converting the obtained compound to its pharmaceutically acceptable salt.

28. A process for the preparation of a compound according to any one of claims 2, 6 to 13, 17, 24 and 25 wherein E is
〉C=C=CHR
comprising the steps of
(a) reducing a 6-chloro-2'-ethynyl-2'-hydroxy-adenosine derivative, bearing appropriate blocking groups, to yield the 6-chloro-2'-ethenylidene-adenosine derivative, bearing appropriate blocking groups,
(b) deblocking the 6-chloro-2'-ethenylidene-adenosine derivative by reacting with acid,
(c) substituting the 6-chloro moiety of the deblocked 6-chloro-2'-ethenylidene-adenosine derivative with the desired moiety as represented by the term "Q" , and optionally
(d) converting the obtained compound to its pharmaceutically acceptable salt.

29. A process for the preparation of a compound according to any one of claims 3, 6 to 13, 18, 19, 26 and 27, wherein E is 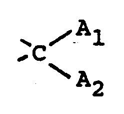 comprising the steps of
(a) reducing a 6-chloro-2'-ethynyl-2'-hydroxy-adenosine derivative, bearing appropriate blocking groups, to yield the 6-chloro-2'-ethynyl-adenosine derivative,
(b) substituting the 6-chloro moiety of the deblocked 6-chloro-2'ethenylidene-adenosine derivative with the desired moiety as represented by the term "Q", and optionally
(c) converting the obtained compound to its pharmaceutically acceptable salt.

30. A compound of any one of claims 1 to 25 or a mixture thereof for use as a pharmaceutically active substance.

31. A compound of any one of claims 1 to 25 or a mixture thereof for use as an inhibitor of the AdoMet-dependent transmethylation activity.

32. A compound of any one of claims 1 to 25 or a mixture thereof for use as an antineoplastic agent.

33. A compound of any one of claims 1 to 25 or a mixture thereof for use as an antiviral agent.

34. A pharmaceutical composition comprising a therapeutically effective amount of a compound of any one of claims 1 to 25 in admixture or otherwise in association with one or more pharmaceutically acceptable carriers or excipients.

35. A pharmaceutical composition comprising a therapeutically effective amount of a compound of any one of claims 1 to 25 wherein Q is NH₂ and a therapeutically effective ADA inhibitory amount of an ADA inhibitor, in admixture or otherwise in association with one or more pharmaceutically acceptable carriers or excipients.

## Claims (Claims for the following Contracting State(s): ES, GR)

1. A compound of the general formula 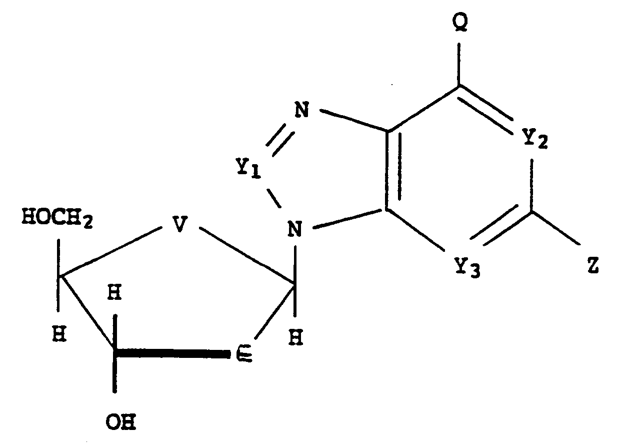 wherein
E is 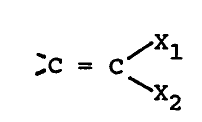 (X₁ and X₂ are each halogens, or one is hydrogen and the other halogen)
〉C = C = CHR (R is hydrogen or C₁-C₄-alkyl), or 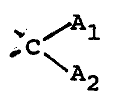 (one of A₁ and A₂ is hydrogen, the other -C≡C-R, wherein R is defined above),
V is oxy, methylene, or thio,
Y₁ is nitrogen, a CH group, a CCl group, a CBr group or a CNH₂ group,
Y₂ and Y₃ are each independently nitrogen or a CH group,
Q is NH₂, NHOH, NHCH₃, or hydrogen, and
Z is hydrogen, halogen, or NH₂;
or a pharmaceutically acceptable salt thereof. or a pharmaceutically acceptable salt thereof.

2. A compound of claim 1 wherein E is 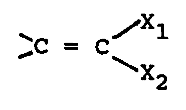 and X₁ is fluoro and X₂ is hydrogen.

3. A compound of claim 1 wherein E is 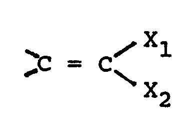 and X₁ is hydrogen and X₂ is fluoro.

4. A compound of claim 1 wherein E is
〉C = C = CHR or 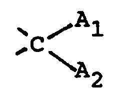 and R is hydrogen.

5. A compound of any one of claims 1 to 4 wherein V is oxy.

6. A compound of any one of claims 1 to 5 wherein Q is NH₂.

7. A compound of any one of Claims 1 to 6 wherein Z is hydrogen.

8. A compound of any one of claims 1 to 7 wherein Y₁ is a CH group.

9. A compound of any one of claims 1 to 8 wherein Y₂ is nitrogen.

10. A compound of any one of claims 1 to 8 wherein Y₂ is a CH group.

11. A compound of any one of claims 1 to 10 wherein Y₃ is nitrogen.

12. The compound of claim 1 wherein the compound is 2'-deoxy-2'-difluoromethylidene-adenosine.

13. The compound of claim 1 wherein the compound is (Z)-2'-deoxy-2'-fluoromethylidene-adenosine.

14. The compound of Claim 1 wherein the compound is (E)-2'-deoxy-2'-fluoromethylidene-adenosine.

15. The compound of claim 1 wherein the compound is 2'-deoxy-2'-ethenylidene-adenosine.

16. The compound of claim 1 wherein the compound is 2'-deoxy-2'(R)-ethynyl-adenosine.

17. The compound of claim 1 wherein the compound is 2'-deoxy-2'(S)-ethynyl-adenosine.

18. The compound of claim 1 wherein the compound is 2'-deoxy-2'-difluoromethylidene-aristeromycin.

19. The compound of claim 1 wherein the compound is 2'-deoxy-2'-difluoromethylidene-3-deaza-adenosine

20. The compound of claim 1 wherein the compound is (E) or (Z)-2'-deoxy-2'-fluoromethylidene-aristeromycin.

21. The compound of claim 1 wherein the compound is (E) or (Z)-2'-deoxy-2'-fluoromethylidene-3-deazaadenosine.

22. The compound of claim 1 wherein the compound is 2'-deoxy-2'-ethenylidene-aristeromycin.

23. The compound of claim 1 wherein the compound is 2'-deoxy-2'-ethenylidene-3-deazaadenosine.

24. The compound of claim 1 wherein the compound is 2'-deoxy-2'(R or S)-ethynyl-aristeromycin.

25. The compound of claim 1 wherein the compound is 2'-deoxy-2'(R or S)-ethynyl-3-deazaadenosine.

26. A process for the preparation of a compound according to any one of claims 1, 5 to 12, 18 and 19, wherein E is 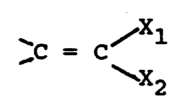 and X₁ and X₂ are each halogens
comprising the steps of
(a) reacting a 6-chloro-2'-keto-adenosine derivative, bearing appropriate blocking groups, with a dihalomethylidene phosphorus ylide to yield a 6-chloro-2'-dihalometylidene-adenosine derivative, bearing appropriate blocking groups,
(b) deblocking the 6-chloro-2'-dihalomethylidene-adenosine derivative by reacting with acid,
(c) substituting the 6-chloro moiety of the deblocked 6-chloro-2'-dihalomethylidene-adenosine derivative with a desired moiety as represented by the term "Q", and optionally
(d) converting to a pharmaceutically acceptable salt thereof.

27. A process for the preparation of a compound according to amy one of claims 1 to 3, 5 to 11, 13, 14, 20 and 21 wherein E is 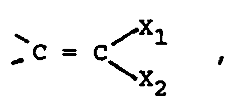 and one of X₁ and X₂ is hydrogen and the other halogen comprising the steps of
(a) reacting a 2'-phenylsulfonyl-halomethylidene adenosine derivative, bearing appropriate blocking groups, with tributyl-tin hydride in the presence of 2,2'-azobis-isobutylnitrile to form the corresponding 2'-tributyl-tin-halomethylidene derivative,
(b) reacting the 2'-tributyl-tin-halomethylidene derivative thus formed with hexamethyldisilazane in the presence of a catalytic amount of ammonia in formamide,
(c) removing the blocking groups, and optionally
(d) converting to a pharmaceutically acceptable salt thereof.

28. A process for the preparation of a compound according to any one of claims 2, 6 to 13, 17, 24 and 25 wherein E is
〉C=C=CHR
comprising the steps of
(a) reducing a 6-chloro-2'-ethynyl-2'-hydroxy-adenosine derivative, bearing appropriate blocking groups, to yield the 6-chloro-2'-ethenylidene-adenosine derivative, bearing appropriate blocking groups,
(b) deblocking the 6-chloro-2'-ethenylidene-adenosine derivative by reacting with acid,
(c) substituting the 6-chloro moiety of the deblocked 6-chloro-2'-ethenylidene-adenosine derivative with the desired moiety as represented by the term "Q" , and optionally
(d) converting to a pharmaceutically acceptable salt thereof.

29. A process for the preparation of a compound according to any one of claims 3, 6 to 13, 18, 19, 26 and 27, wherein E is 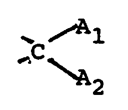 comprising the steps of
(a) reducing a 6-chloro-2'-ethynyl-2'-hydroxy-adenosine derivative, bearing appropriate blocking groups, to yield the 6-chloro-2'-ethynyl-adenosine derivative,
(b) substituting the 6-chloro moiety of the deblocked 6-chloro-2'-ethenylidene-adenosine derivative with the desired moiety as represented by the term "Q", and optionally
(c) converting to a pharmaceutically acceptable salt thereof.

30. A method for the preparation of a pharmaceutical composition comprising combining a compound according to any one of claims 1 to 25 with a pharmaceutically acceptable carrier.

31. A method according to claim 30 wherein the pharmaceutical composition prepared is for use as an inhibitor of the AdoMet-dependent transmethylation activity.

32. A method according to claim 30 wherein the pharmaceutical composition prepared is for use as an antineoplastic agent.

33. A method according to claim 30 wherein the pharmaceutical composition prepared is for use as an antiviral agent.

## Patentansprüche (Patentansprüche für folgende(n) Vertragsstaat(en): AT, BE, CH, DE, FR, GB, IT, LI, LU, NL, SE)

1. Verbindung der allgemeinen Formel 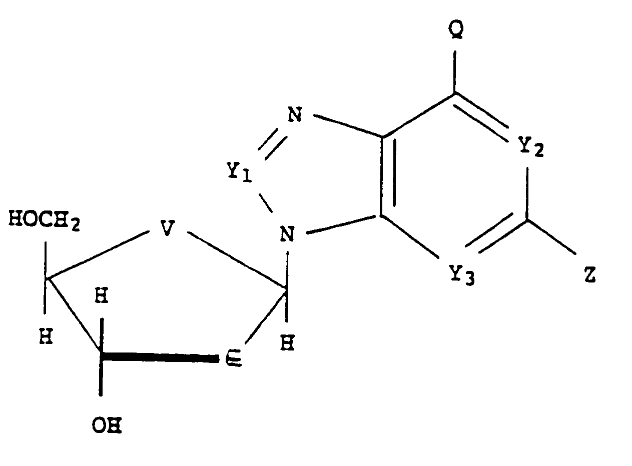 in der
E für 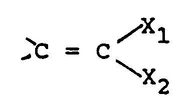 (X₁ und X₂ sind jeweils Halogenatome oder eines ist ein Wasserstoff- und das andere ein Halogenatom)
〉C = C = CHR (R ist ein Wasserstoffatom oder ein C₁-C₄-Alkylrest) oder 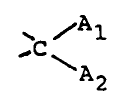 (eines von A₁ und A₂ ist ein Wasserstoffatom, das andere -C≡C-R, wobei R die vorstehend angegebene Bedeutung hat), steht,
V für eine Oxy-, Methylen- oder Thiogruppe steht,
Y₁ ein Stickstoffatom, eine CH-Gruppe, eine CCl-Gruppe, eine CBr-Gruppe oder eine CNH₂-Gruppe ist,
Y₂ und Y₃ jeweils unabhängig voneinander für ein Stickstoffatom oder eine CH-Gruppe stehen,
Q eine NH₂-, NHOH-, NHCH₃-Gruppe oder ein Wasserstoffatom bedeutet, und
Z für ein Wasserstoffatom, ein Halogenatom oder eine NH₂-Gruppe steht;
oder ein pharmazeutisch verträgliches Salz davon.

2. Verbindung nach Anspruch 1, in der E für 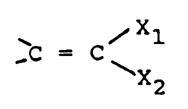 steht und X₁ ein Fluoratom ist und X₂ ein Wasserstoffatom ist.

3. Verbindung nach Anspruch 1, in der E für 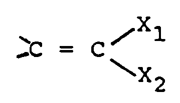 steht und X₁ ein Wasserstoffatom ist und X₂ ein Fluoratom ist.

4. Verbindung nach Anspruch 1, in der E für
〉C = C = CHR oder 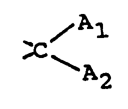 steht und R ein Wasserstoffatom ist.

5. Verbindung nach einem der Ansprüche 1 bis 4, in der V für eine Oxy-Gruppe steht.

6. Verbindung nach einem der Ansprüche 1 bis 5, in der Q für eine NH₂-Gruppe steht.

7. Verbindung nach einem der Ansprüche 1 bis 6, in der Z ein Wasserstoffatom ist.

8. Verbindung nach einem der Ansprüche 1 bis 7, in der Y₁ für eine CH-Gruppe steht.

9. Verbindung nach einem der Ansprüche 1 bis 8, in der Y₂ ein Stickstoffatom ist.

10. Verbindung nach einem der Ansprüche 1 bis 8, in der Y₂ eine CH-Gruppe ist.

11. Verbindung nach einem der Ansprüche 1 bis 10, in der Y₃ ein Stickstoffatom ist.

12. Verbindung nach Anspruch 1, wobei die Verbindung 2'-Desoxy-2'-difluormethyliden-adenosin ist.

13. Verbindung nach Anspruch 1, wobei die Verbindung (Z)-2'-Desoxy-2'-fluormethyliden-adenosin ist.

14. Verbindung nach Anspruch 1, wobei die Verbindung (E)-2'-Desoxy-2'-fluormethyliden-adenosin ist.

15. Verbindung nach Anspruch 1, wobei die Verbindung 2'-Desoxy-2'-ethenyliden-adenosin ist.

16. Verbindung nach Anspruch 1, wobei die Verbindung 2'-Desoxy-2'(R)-ethinyl-adenosin ist.

17. Verbindung nach Anspruch 1, wobei die Verbindung 2'-Desoxy-2'(S)-ethinyl-adenosin ist.

18. Verbindung nach Anspruch 1, wobei die Verbindung 2'-Desoxy-2'-difluormethyliden-aristeromycin ist.

19. Verbindung nach Anspruch 1, wobei die Verbindung 2'-Desoxy-2'-difluormethyliden-3-deazaadenosin ist.

20. Verbindung nach Anspruch 1, wobei die Verbindung (E)- oder (Z)-2'-Desoxy-2'-fluormethyliden-aristeromycin ist.

21. Verbindung nach Anspruch 1, wobei die Verbindung (E)- oder (Z)-2'-Desoxy-2'-fluormethyliden-3-deazaadenosin ist.

22. Verbindung nach Anspruch 1, wobei die Verbindung 2'-Desoxy-2'-ethenyliden-aristeromycin ist.

23. Verbindung nach Anspruch 1, wobei die Verbindung 2'-Desoxy-2'-ethenyliden-3-deazaadenosin ist.

24. Verbindung nach Anspruch 1, wobei die Verbindung 2'-Desoxy-2'(R oder S)-ethinyl-aristeromycin ist.

25. Verbindung nach Anspruch 1, wobei die Verbindung 2'-Desoxy-2'(R oder S)-ethinyl-3-deazaadenosin ist.

26. Verfahren zur Herstellung einer Verbindung nach einem der Ansprüche 1, 5 bis 12, 18 und 19, wobei E für 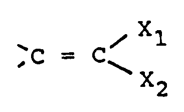 steht und X₁ und X₂ jeweils Halogenatome sind,
umfassend die Schritte
(a) Umsetzen eines 6-Chlor-2'-keto-adenosin-Derivates, das geeignete Blockierungsgruppen trägt, mit einem Dihalogenmethyliden-phosphorylid, wobei ein 6-Chlor-2'-dihalogenmethyliden-adenosin-Derivat, das geeignete Blockierungsgruppen trägt, erhalten wird,
(b) Entblockieren des 6-Chlor-2'-dihalogenmethyliden-adenosin-Derivates durch Umsetzen mit Säure,
(c) Substituieren der 6-Chlor-Einheit des entblockierten 6-Chlor-2'-dihalogenmethyliden-adenosin-Derivates mit einer gewünschten Einheit, wie sie durch das Kürzel "Q" repräsentiert wird, und gegebenenfalls
(d) Umwandeln der erhaltenen Verbindung in ihr pharmazeutisch verträgliches Salz.

27. Verfahren zur Herstellung einer Verbindung nach einem der Ansprüche 1 bis 3, 5 bis 11, 13, 14, 20 und 21, wobei E für 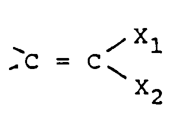 steht und eines von X₁ und X₂ ein Wasserstoffatom und das andere ein Halogenatom ist,
umfassend die Schritte
(a) Umsetzen eines 2'-Phenylsulfonyl-halogenmethyliden-adenosin-Derivates, das geeignete Blockierungsgruppen trägt, mit Tributylzinnhydrid in Gegenwart von 2,2'-Azobis-isobutylnitril, um das entsprechende 2'-Tributylzinn-halogenmethyliden-Derivat zu erzeugen,
(b) Umsetzen des so erzeugten 2'-Tributylzinn-halogenmethyllden-Derivates mit Hexamethyldisilazan in Gegenwart einer katalytischen Menge von Ammoniak in Formamid,
(c) Entfernen der Blockierungsgruppe, und gegebenenfalls
(d) Umwandeln der erhaltenen Verbindung in ihr pharmazeutisch verträgliches Salz.

28. Verfahren zur Herstellung einer Verbindung nach einem der Ansprüche 2, 6 bis 13, 17, 24 und 25, wobei E für
C = C = CHR
steht, umfassend die Schritte
(a) Reduzieren eines 6-Chlor-2'-ethinyl-2'-hydroxy-adenosin-Derivates, das geeignete Blockierungsgruppen trägt, wobei das 6-Chlor-2'-ethenyliden-adenosin-Derivat, das geeignete Blockierungsgruppen trägt, erhalten wird,
(b) Entblockieren des 6-Chlor-2'-ethenyliden-adenosin-Derivates durch Umsetzen mit Säure,
(c) Substituieren der 6-Chlor-Einheit des entblockierten 6-Chlor-2'-ethenyliden-adenosin-Derivates mit der gewünschten Einheit, wie sie durch das Kürzel "Q" repräsentiert wird, und gegebenenfalls
(d) Umwandeln der erhaltenen Verbindung in ihr pharmazeutisch verträgliches Salz.

29. Verfahren zur Herstellung einer Verbindung nach einem der Ansprüche 3, 6 bis 13, 18, 19, 26 und 27, wobei E für 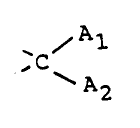 steht, umfassend die Schritte
(a) Reduzieren eines 6-Chlor-2'-ethinyl-2'-hydroxy-adenosin-Derivates, das geeignete Blockierungsgruppen trägt, wobei das 6-Chlor-2'-ethinyl-adenosin-Derivat erhalten wird,
(b) Substituieren der 6-Chlor-Einheit des entblockierten 6-Chlor-2'-ethenyliden-adenosin-Derivates mit der gewünschten Einheit, wie sie durch das Kürzel "Q" repräsentiert wird, und gegebenenfalls
(c) Umwandeln der erhaltenen Verbindung in ihr pharmazeutisch verträgliches Salz.

30. Verbindung nach einem der Ansprüche 1 bis 25 oder ein Gemisch davon zur Verwendung als pharmazeutischen Wirkstoff.

31. Verbindung nach einem der Ansprüche 1 bis 25 oder ein Gemisch davon zur Verwendung als Inhibitor der AdoMet-abhängigen Transmethylierungsaktivität.

32. Verbindung nach einem der Ansprüche 1 bis 25 oder ein Gemisch davon zur Verwendung als anti-neoplastisches Mittel.

33. Verbindung nach einem der Ansprüche 1 bis 25 oder ein Gemisch davon zur Verwendung als anti-virales Mittel.

34. Ein Arzneimittel, umfassend eine therapeutisch wirksame Menge einer Verbindung nach einem der Ansprüche 1 bis 25 im Gemisch oder anderenfalls in Verbindung mit einem oder mehreren pharmazeutisch verträglichen Trägern oder Excipienten.

35. Ein Arzneimittel, umfassend eine therapeutisch wirksame Menge einer Verbindung nach einem der Ansprüche 1 bis 25, in der Q eine NH₂-Gruppe ist, und eine therapeutisch wirksame ADA-inhibierende Menge eines ADA-Inhibitors im Gemisch oder anderenfalls in Verbindung mit einem oder mehreren pharmazeutisch verträglichen Trägern oder Excipienten.

## Patentansprüche (Patentansprüche für folgende(n) Vertragsstaat(en): ES, GR)

1. Verbindung der allgemeinen Formel 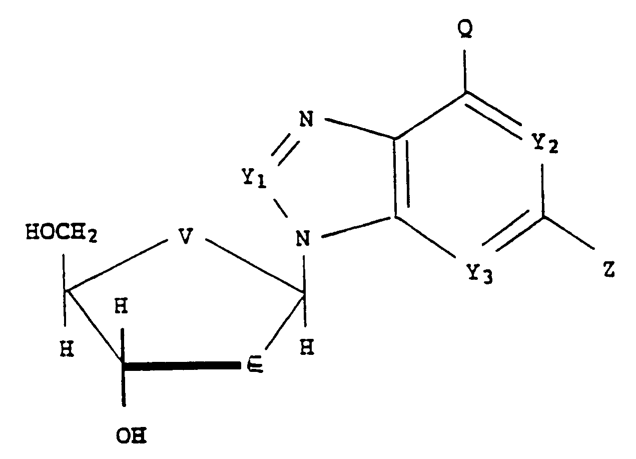 in der
E für 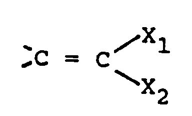 (X₁ und X₂ sind jeweils Halogenatome oder eines ist ein Wasserstoff- und das andere ein Halogenatom)
〉C = C = CHR (R ist ein Wasserstoffatom oder ein C₁-C₄-Alkylrest) oder 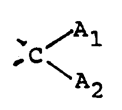 (eines von A₁ und A₂ ist ein Wasserstoffatom, das andere -C≡C-R, wobei R die vorstehend angegebene Bedeutung hat), steht,
V für eine Oxy-, Methylen- oder Thiogruppe steht,
Y₁ ein Stickstoffatom, eine CH-Gruppe, eine CCl-Gruppe, eine CBr-Gruppe oder eine CNH₂-Gruppe ist,
Y₂ und Y₃ jeweils unabhängig voneinander für ein Stickstoffatom oder eine CH-Gruppe stehen,
Q eine NH₂-, NHOH-, NHCH₃-Gruppe oder ein Wasserstoffatom bedeutet, und
Z für ein Wasserstoffatom, ein Halogenatom oder eine NH₂-Gruppe steht;
oder ein pharmazeutisch verträgliches Salz davon.

2. Verbindung nach Anspruch 1, in der E für 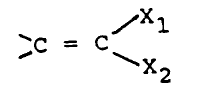 steht und X₁ ein Fluoratom ist und X₂ ein Wasserstoffatom ist.

3. Verbindung nach Anspruch 1, in der E für 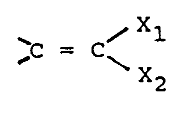 steht und X₁ ein Wasserstoffatom ist und X₂ ein Fluoratom ist.

4. Verbindung nach Anspruch 1, in der E für
〉C = C = CHR oder 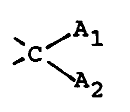 steht und R ein Wasserstoffatom ist.

5. Verbindung nach einem der Ansprüche 1 bis 4, in der V für eine Oxy-Gruppe steht.

6. Verbindung nach einem der Ansprüche 1 bis 5, in der Q für eine NH₂-Gruppe steht.

7. Verbindung nach einem der Ansprüche 1 bis 6, in der Z ein Wasserstoffatom ist.

8. Verbindung nach einem der Ansprüche 1 bis 7, in der Y₁ für eine CH-Gruppe steht.

9. Verbindung nach einem der Ansprüche 1 bis 8, in der Y₂ ein Stickstoffatom ist.

10. Verbindung nach einem der Ansprüche 1 bis 8, in der Y₂ eine CH-Gruppe ist.

11. Verbindung nach einem der Ansprüche 1 bis 10, in der Y₃ ein Stickstoffatom ist.

12. Verbindung nach Anspruch 1, wobei die Verbindung 2'-Desoxy-2'-difluormethyliden-adenosin ist.

13. Verbindung nach Anspruch 1, wobei die Verbindung (Z)-2'-Desoxy-2'-fluormethyliden-adenosin ist.

14. Verbindung nach Anspruch 1, wobei die Verbindung (E)-2'-Desoxy-2'-fluormethyliden-adenosin ist.

15. Verbindung nach Anspruch 1, wobei die Verbindung 2'-Desoxy-2'-ethenyliden-adenosin ist.

16. Verbindung nach Anspruch 1, wobei die Verbindung 2'-Desoxy-2'(R)-ethinyl-adenosin ist.

17. Verbindung nach Anspruch 1, wobei die Verbindung 2'-Desoxy-2'(S)-ethinyl-adenosin ist.

18. Verbindung nach Anspruch 1, wobei die Verbindung 2'-Desoxy-2'-difluormethyliden-aristeromycin ist.

19. Verbindung nach Anspruch 1, wobei die Verbindung 2'-Desoxy-2'-difluormethyliden-3-deazaadenosin ist.

20. Verbindung nach Anspruch 1, wobei die Verbindung (E)- oder (Z)-2'-Desoxy-2'-fluormethyliden-aristeromycin ist.

21. Verbindung nach Anspruch 1, wobei die Verbindung (E)- oder (Z)-2'-Desoxy-2'-fluormethyliden-3-deazaadenosin ist.

22. Verbindung nach Anspruch 1, wobei die Verbindung 2'-Desoxy-2'-ethenyliden-aristeromycin ist.

23. Verbindung nach Anspruch 1, wobei die Verbindung 2'-Desoxy-2'-ethenyliden-3-deazaadenosin ist.

24. Verbindung nach Anspruch 1, wobei die Verbindung 2'-Desoxy-2'(R oder S)-ethinyl-aristeromycin ist.

25. Verbindung nach Anspruch 1, wobei die Verbindung 2'-Desoxy-2'(R oder S)-ethinyl-3-deazaadenosin ist.

26. Verfahren zur Herstellung einer Verbindung nach einem der Ansprüche 1, 5 bis 12, 18 und 19, wobei E für 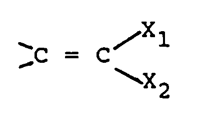 steht und X₁ und X₂ jeweils Halogenatome sind,
umfassend die Schritte
(a) Umsetzen eines 6-Chlor-2'-keto-adenosin-Derivates, das geeignete Blockierungsgruppen trägt, mit einem Dihalogenmethyliden-phosphorylid, wobei ein 6-Chlor-2'-dihalogenmethyliden-adenosin-Derivat, das geeignete Blockierungsgruppen trägt, erhalten wird,
(b) Entblockieren des 6-Chlor-2'-dihalogenmethyliden-adenosin-Derivates durch Umsetzen mit Säure,
(c) Substituieren der 6-Chlor-Einheit des entblockierten 6-Chlor-2'-dihalogenmethyliden-adenosin-Derivates mit einer gewünschten Einheit, wie sie durch das Kürzel "Q" repräsentiert wird, und gegebenenfalls
(d) Umwandeln in ein pharmazeutisch verträgliches Salz davon.

27. Verfahren zur Herstellung einer Verbindung nach einem der Ansprüche 1 bis 3, 5 bis 11, 13, 14, 20 und 21, wobei E für 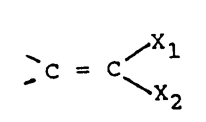 steht und eines von X₁ und X₂ ein Wasserstoffatom und das andere ein Halogenatom ist,
umfassend die Schritte
(a) Umsetzen eines 2'-Phenylsulfonyl-halogenmethyliden-adenosin-Derivates, das geeignete Blockierungsgruppen trägt, mit Tributylzinnhydrid in Gegenwart von 2,2'-Azobis-isobutylnitril, um das entsprechende 2'-Tributylzinn-halogenmethyliden-Derivat zu erzeugen,
(b) Umsetzen des so erzeugten 2'-Tributylzinn-halogenmethyliden-Derivates mit Hexamethyldisilazan in Gegenwart einer katalytischen Menge von Ammoniak in Formamid,
(c) Entfernen der Blockierungsgruppen, und gegebenenfalls
(d) Umwandeln in ein pharmazeutisch verträgliches Salz davon.

28. Verfahren zur Herstellung einer Verbindung nach einem der Ansprüche 2, 6 bis 13, 17, 24 und 25, wobei E für
C = C = CHR
steht, umfassend die Schritte
(a) Reduzieren eines 6-Chlor-2'-ethinyl-2'-hydroxy-adenosin-Derivates, das geeignete Blockierungsgruppen trägt, wobei das 6-Chlor-2'-ethenyliden-adenosin-Derivat, das geeignete Blockierungsgruppen trägt, erhalten wird,
(b) Entblockieren des 6-Chlor-2'-ethenyliden-adenosin-Derivates durch Umsetzen mit Säure,
(c) Substituieren der 6-Chlor-Einheit des entblockierten 6-Chlor-2'-ethenyliden-adenosin-Derivates mit der gewünschten Einheit, wie sie durch das Kürzel "Q" repräsentiert wird, und gegebenenfalls
(d) Umwandeln in ein pharmazeutisch verträgliches Salz davon.

29. Verfahren zur Herstellung einer Verbindung nach einem der Ansprüche 3, 6 bis 13, 18, 19, 26 und 27, wobei E für 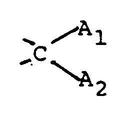 steht, umfassend die Schritte
(a) Reduzieren eines 6-Chlor-2'-ethinyl-2'-hydroxy-adenosin-Derivates, das geeignete Blockierungsgruppen trägt, wobei das 6-Chlor-2'-ethinyl-adenosin-Derivat erhalten wird,
(b) Substituieren der 6-Chlor-Einheit des entblockierten 6-Chlor-2'-ethenyliden-adenosin-Derivates mit der gewünschten Einheit, wie sie durch das Kürzel "Q" repräsentiert wird, und gegebenenfalls
(c) Umwandeln in ein pharmazeutisch verträgliches Salz davon.

30. Verfahren zur Herstellung eines Arzneimittels, umfassend das Vereinigen einer Verbindung nach einem der Ansprüche 1 bis 25 mit einem pharmazeutisch verträglichen Träger.

31. Verfahren nach Anspruch 30, wobei das hergestellte Arzneimittel zur Verwendung als Inhibitor der AdoMet-abhängigen Transmethylierungsaktivität vorgesehen ist.

32. Verfahren nach Anspruch 30, wobei das hergestellte Arzneimittel zur Verwendung als anti-neoplastisches Mittel vorgesehen ist.

33. Verfahren nach Anspruch 30, wobei das hergestellte Arzneimittel zur Verwendung als anti-virales Mittel vorgesehen ist.

## Revendications (Revendications pour l'(les) Etat(s) contractant(s) suivant(s): AT, BE, CH, DE, FR, GB, IT, LI, LU, NL, SE)

1. Un composé de formule générale : 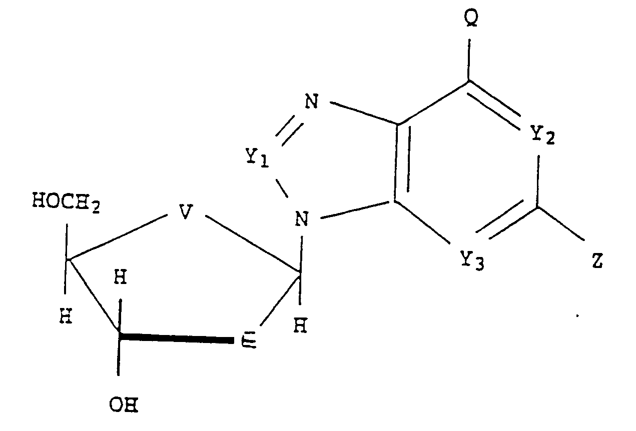 dans laquelle
E est 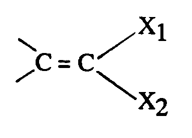 (X₁ et X₂ désignent chacun des atomes d'halogène, ou bien l'un est un atome d'hydrogène et l'autre un atome d'halogène)
〉C = C = CHR (R est un atome d'hydrogène ou un groupe C₁-C₄ alkyle), ou bien 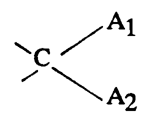 (l'un des A₁ et A₂ est un atome d'hydrogène et l'autre un groupe -C≡C-R, où R est défini comme précédemment),
V est un groupe oxy, méthylène ou thio,
Y₁ est un atome d'azote, un groupe CH, CCl, CBr ou CNH₂,
Y₂ et Y₃ désignent chacun indépendamment un atome d'azote ou un groupe CH,
Q est un groupe NH₂, NHOH, NHCH₃, ou un atome d'hydrogène, et
Z est un atome d'hyrogène, d'halogène ou a groupe NH₂;
ou bien un sel pharmaceutiquement acceptable dudit composé.

2. Un composé selon la revendication 1, dans laquelle E est 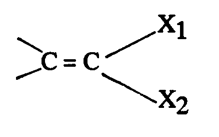 et X₁ est un coupe fluoro et X₂ est un atome d'hydrogène.

3. Un composé selon la revendication 1, selon laquelle E est 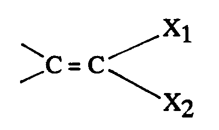 et X₁ est un atome d'hydrogène et X₂ est un groupe fluoro.

4. Un composé selon la revendication 1, selon laquelle E est
C = C = CHR ou 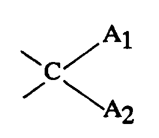 et R est un atome d'hydrogène.

5. Un composé selon l'une quelconque des revendications 1 à 4, selon laquelle V est un groupe oxy.

6. Un composé selon l'une quelconque des revendications 1 à 5, selon laquelle Q est un coupe NH₂.

7. Un composé selon l'une quelconque des revendications 1 à 6, selon laquelle Z est un atome d'hydrogène.

8. Un composé selon l'une quelconque des revendications 1 à 7, selon laquelle Y₁ est un groupe CH.

9. Un composé selon l'une quelconque des revendications 1 à 8, selon laquelle Y₂ est un atome d'azote.

10. Un composé selon l'une quelconque des revendications 1 à 8, selon laquelle Y₂ est un groupe CH.

11. Un composé selon l'une quelconque des revendications 1 à 10, selon laquelle Y₃ est un atome d'azote.

12. Le composé selon la revendication 1, selon laquelle le composé est la 2'-désoxy-2'-difluorométhylidène-adénosine.

13. Le composé selon la revendication 1, selon laquelle le composé est la (Z)-2'-désoxy-2'- fluorométhylidène-adénosine.

14. Le composé selon la revendication 1, selon laquelle le composé est la (E)-2'-désoxy-2'- fluorométhylidène-adénosine.

15. Le composé selon la revendication 1, selon laquelle le composé est la 2'-désoxy-2'-éthénylidène-adénosine.

16. Le composé selon la revendication 1, selon laquelle le composé est la 2'-désoxy-2'-(R)-éthynyl-adénosine.

17. Le composé selon la revendication 1, selon laquelle le composé est la 2'-désoxy-2'(S)-éthynyl-adénosine.

18. Le composé selon la revendication 1, selon laquelle le composé est la 2'-désoxy-2'-difluorométhylidène-aristéromycine.

19. Le composé selon la revendication 1, selon laquelle le composé est la 2'-désoxy-2'-difluorométhylidène-3-déaza-adénosine.

20. Le composé selon la revendication 1, selon laquelle le composé est (E)ou (Z)-2'-désoxy-2'- fluorométhylidène-aristéromycine.

21. Le composé selon la revendication 1, selon laquelle le composé est (E)ou (Z)-2'-désoxy-2'- fluorométhylidène-3-déazaadénosine.

22. Le composé selon la revendication 1, selon laquelle le composé est la 2'-désoxy-2'-éthénylidène-aristéromycine.

23. Le composé selon la revendication 1, selon laquelle le composé est la 2'-désoxy-2'-éthénylidène-3-déazaadénosine.

24. Le composé selon la revendication 1, selon laquelle le composé est la 2'-désoxy-2' (R ou S)-éthynyl-aristéromycine.

25. Le composé selon la revendication 1, selon laquelle le composé est la 2'-désoxy-2' (R ou S)-éthynyl-3-déazaadénosine.

26. Un procédé pour la préparation d'un composé selon l'une quelconque des revendications 1, 5 à 12, 18 et 19 selon laquelle E est 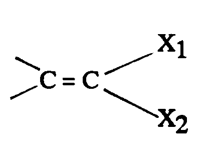 et X₁ et X₂ désigent chacun des atomes d'halogène, comprenant les étapes suivantes :
(a) réaction d'un dérivé 6-chloro-2'-céto-adénosine, portant les groupes bloquants appropriés, avec un dihalométhylure de phosphore pour donner un dérivé 6-chloro-2'-dihalométhylidène-adénosine, portant les groupes bloquants appropriés,
(b) déblocage du dérivé 6-chloro-2'-dihalométhylidène-adénosine par réaction avec un acide,
(c) substitution de la portion 6-chloro du dérivé 6-chloro-2'-dihalomethylidène-adénosine débloqué avec une portion désirée représentée par le terme "Q", et éventuellement
(d) conversion du composé obtenu en son sel pharmaceutiquement acceptable.

27. Un procédé de préparation d'un composé selon l'une quelconque des revendications 1 à 3, 5 à 11, 13, 14, 20 et 21, selon laquelle E est 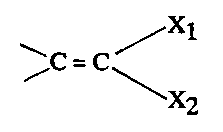 et l'un des X₁ et X₂ est un atome d'hydrogène et l'autre un atome d'halogène comprenant les étapes suivantes :
(a) réaction d'un dérivé 2'-phénylsulfonyl-halométhylidène-adénosine, portant les groupes bloquants appropriés, avec l'hydrure de tributyl-étain en présence de 2,2'-azobisisobutylnitrile pour former le dérivé 2'-tributyl-étain-halométhylidène correspondant,
(b) réaction du dérivé 2'-tributyl-étain-halométhylidène ainsi formé avec le hexaméthyldisilazane en présence d'une quantité catalytique d'ammoniac dans du formamide,
(c) élimination des groupes bloquants, et éventuellement
(d) conversion du composé obtenu en son sel pharmaceutiquement acceptable.

28. Un procédé de préparation d'un composé selon l'une quelconque des revendications 2, 6 à 13, 17, 24 et 25, selon laquelle E est
〉C=C=CHR
comprenant les étapes suivantes :
(a) réduction d'un dérivé 6-chloro-2'-éthynyl-2'-hydroxy-adénosine, portant les groupes bloquants appropriés, pour donner le dérivé 6-chloro-2'-éthénylidène-adénosine, portant les groupes bloquants appropriés,
(b) déblocage du dérivé 6-chloro-2'-éthénylidène-adénosine par réaction avec un acide,
(c) substitution de la portion 6-chloro du dérivé 6-chloro-2'-éthénylidène-adénosine débloqué par la portion désirée représentée par le terme "Q", et éventuellement
(d) conversion du composé obtenu en son sel pharmaceutiquement acceptable.

29. Un procédé de préparation d'un composé selon l'une quelconque des revendications 3, 6 à 13, 18, 19, 26 et 27, selon laquelle E est 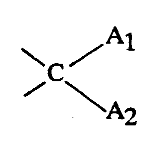 comprenant les étapes suivantes :
(a) réduction d'un dérivé 6-chloro-2'-éthynyl-2'-hydroxy-adénosine, portant les groupes bloquants appropriés, pour donner le dérivé 6-chloro-2'-éthynyl-adénosine,
(b) substitution de la portion 6-chloro du dérivé 6-chloro-2'-éthénylidène-adénosine débloqué par la portion désiré représentée par le terme "Q", et éventuellement
(c) conversion du composé obtenu en son sel pharmaceutiquement acceptable.

30. Un composé selon l'une quelconque des revendications 1 à 25 ou un mélange de ces composés à utiliser comme substance pharmaceutiquement active.

31. Un composé selon l'une quelconque des revendications 1 à 25 ou un mélange de ces composés à utiliser comme inhibiteur de l'activité de transmé-thylation dépendant de l'AdoMet.

32. Un composé selon l'une quelconque des revendications 1 à 25 ou un mélange de ces composés à utiliser comme agent antinéoplastique.

33. Un composé selon l'une quelconque des revendications 1 à 25 ou un mélange de ces composés à utiliser comme agent antiviral.

34. Une composition pharmaceutique comprenant une quantité thérapeutiquement active d'un composé selon l'une quelconque des revendications 1 à 25 en mélange ou en association avec un ou plusieurs supports ou excipients pharmaceutiquement acceptables.

35. Une composition pharmaceutique comprenant une quantité thérapeutiquement active d'un composé selon l'une quelconque des revendications 1 à 25 selon laquelle Q est NH₂ et une quantité inhibitrice de ADA thérapeutiquement efficace d'un inhibiteur de ADA, en mélange ou en association avec un ou plusieurs supports ou excipients pharmaceutiquement acceptables.

## Revendications (Revendications pour l'(les) Etat(s) contractant(s) suivant(s): ES, GR)

1. Un composé de formule générale : 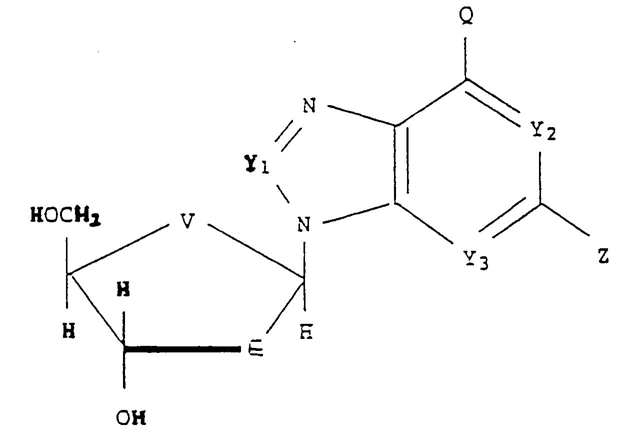 dans laquelle
E est 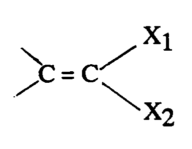 (X₁ et X₂ désignent chacun des atomes d'halogène, ou bien l'un est un atome d'hydrogène et l'autre un atome d'halogène)
〉C=C=CHR (R est un atome d'hydrogène ou un groupe C₁-C₄ alkyle), ou bien 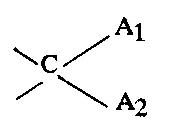 (l'un des A₁ et A₂ est un atome d'hydrogène et l'autre un groupe -C≡C-R, où R est défini comme précédemment),
V est un groupe oxy, méthylène ou thio,
Y₁ est un atome d'azote, un coupe CH, CCl, CBr ou CNH₂,
Y₂ et Y₃ désignent chacun indépendamment un atome d'azote ou un groupe CH,
Q est un groupe NH₂, NHOH, NHCH₃, ou un atome d'hydrogène, et
Z est un atome d'hydrogène, d'halogène ou un groupe NH₂;
ou bien un sel pharmaceutiquement acceptable dudit composé.

2. Un composé selon la revendication 1, dans laquelle E est 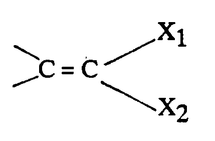 et X₁ est un groupe fluoro et X₂ est un atome d'hydrogène.

3. Un composé selon la revendication 1, selon laquelle E est 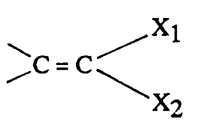 et X₁ est un atome d'hydrogène et X₂ est un groupe fluoro.

4. Un composé selon la revendication 1, selon laquelle E est
C = C = CHR ou 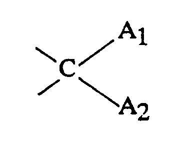 et R est un atome d'hydrogène.

5. Un composé selon l'une quelconque des revendications 1 à 4, selon laquelle V est un groupe oxy.

6. Un composé selon l'une quelconque des revendications 1 à 5, selon laquelle Q est un groupe NH₂.

7. Un composé selon l'une quelconque des revendications 1 à 6, selon laquelle Z est un atome d'hydrogène.

8. Un composé selon l'une quelconque des revendications 1 à 7, selon laquelle Y₁ est un groupe CH.

9. Un composé selon l'une quelconque des revendications 1 à 8, selon laquelle Y₂ est un atome d'azote.

10. Un composé selon l'une quelconque des revendications 1 à 8, selon laquelle Y₂ est un groupe CH.

11. Un composé selon l'une quelconque des revendications 1 à 10, selon laquelle Y₃ est un atome d'azote.

12. Le composé selon la revendication 1, selon laquelle le composé est la 2'-désoxy-2'-difluorométhylidène-adénosine.

13. Le composé selon la revendication 1, selon laquelle le composé est la (Z)-2'-désoxy-2'- fluorométhylidène-adénosine.

14. Le composé selon la revendication 1, selon laquelle le composé est la (E)-2'-désoxy-2'- fluorométhylidène-adénosine.

15. Le composé selon la revendication 1, selon laquelle le composé est la 2'-désoxy-2'-éthénylidène-adénosine.

16. Le composé selon la revendication 1, selon laquelle le composé est la 2'-désoxy-2'-(R)-éthynyl-adénosine.

17. Le composé selon la revendication 1, selon laquelle le composé est la 2'-désoxy-2'(S)-éthynyl-adénosine.

18. Le composé selon la revendication 1, selon laquelle le composé est la 2'-désoxy-2'-difluorométhylidène-aristéromycine.

19. Le composé selon la revendication 1, selon laquelle le composé est la 2'-désoxy-2'-difluorométhylidène-3-déaza-adénosine.

20. Le composé selon la revendication 1, selon laquelle le composé est (E)ou (Z)-2'-désoxy-2'- fluorométhylidène-aristéromycine.

21. Le composé selon la revendication 1, selon laquelle le composé est (E)ou (Z)-2'-désoxy-2'- fluorométhylidène-3-déazaadénosine.

22. Le composé selon la revendication 1, selon laquelle le composé est la 2'-désoxy-2'-éthénylidène-aristéromycine.

23. Le composé selon la revendication 1, selon laquelle le composé est la 2'-désoxy-2'-éthénylidène-3-déazaadénosine.

24. Le composé selon la revendication 1, selon laquelle le composé est la 2'-désoxy-2' (R ou S)-éthynyl-aristéromycine.

25. Le composé selon la revendication 1, selon laquelle le composé est la 2'-désoxy-2' (R ou S)-éthynyl- 3-déazaadénosine.

26. Un procédé pour la préparation d'un composé selon l'une quelconque des revendications 1, 5 à 12, 18 et 19 selon laquelle E est 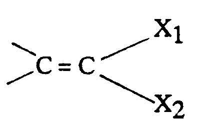 et X₁ et X₂ désirent chacun des atomes d'halogène, comprenant les étapes suivantes :
(a) réaction d'un dérivé 6-chloro-2'-céto-adénosine, portant les groupes bloquants appropriés, avec un dihalométhylure de phosphore pour donner un dérivé 6-chloro-2'-dihalométhylidène-adénosine, portant les groupes bloquants appropriés,
(b) déblocage du dérivé 6-chloro-2'-dihalométhylidène-adénosine par réaction avec un acide,
(c) substitution de la portion 6-chloro du dérivé 6-chloro-2'-dihalométhylidène-adénosine débloqué avec une portion désirée représentée par le terme "Q", et éventuellement
(d) conversion du composé obtenu en son sel pharmaceutiquement acceptable.

27. Un procédé de préparation d'un composé selon l'une quelconque des revendications 1 à 3,5 à 11,13, 14,20 et 21, selon laquelle E est 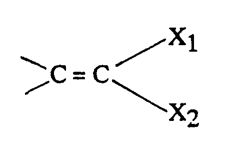 et l'un des X₁ et X₂ est un atome d'hydrogène et l'autre un atome d'halogène comprenant les étapes suivantes :
(a) re'action d'un dérivé 2'-phénylsulfonyl-halométhylidène-adénosine, portant les ,groupes bloquants appropriés, avec l'hydrure de tributyl-étain en présence de 2,2'-azobisisobutylnitrile pour former le dérivé 2'-tributyl-étain-halométhylidène correspondant,
(b) réaction du dérivé 2'-tributyl-étain-halométhylidène ainsi formé avec le hexaméthyldisilazane en présence d'une quantité catalytique d'ammoniac dans du formamide,
(c) élimination des groupes bloquants, et éventuellement
(d) conversion du composé obtenu en son sel pharmaceutiquement acceptable.

28. Un procédé de préparation d'un composé selon l'une quelconque des revendications 2, 6 à 13, 17, 24 et 25, selon laquelle E est
〉C=C=CHR
comprenant les étapes suivantes :
(a) réduction d'un dérivé 6-chloro-2'-éthynyl-2'-hydroxy-adénosine, portant les groupes bloquants appropriés, pour donner le dérivé 6-chloro-2'-éthénylidène-adénosine, portant les groupes bloquants appropriés,
(b) déblocage du dérivé 6-chloro-2'-éthénylidène-adénosine par réaction avec un acide,
(c) substitution de la portion 6-chloro du dérivé 6-chloro-2'-éthénylidène-adénosine débloqué par la portion désirée représentée par le terme "Q", et éventuellement
(d) conversion du composé obtenu en son sel pharmaceutiquement acceptable.

29. Un procédé de préparation d'un composé selon l'une quelconque des revendications 3, 6 à 13, 18, 19, 26 et 27, selon laquelle E est 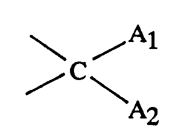 comprenant les étapes suivantes :
(a) réduction d'un dérivé 6-chloro-2'-éthynyl-2'-hydroxy-adénosine, portant les groupes bloquants appropriés, pour donner le dérivé 6-chloro-2'-éthynyl-adénosine,
(b) substitution de la portion 6-chloro du dérivé 6-chloro-2'-éthénylidène-adénosine débloqué par la portion désiré représentée par le terme "Q", et éventuellement
(c) conversion du composé obtenu en son sel pharmaceutiquement acceptable.

30. Un procédé de préparation d'une composition pharmaceutique comprenant la combinaison d'un composé selon l'une quelconque des revendications 1 à 25 avec un support pharmaceutiquement acceptable.

31. Un procédé selon la revendication 30 selon laquelle la composition pharmaceutique préparée est à utiliser comme un inhibiteur de l'activité de transméthylation dépendant de l'AdoMet.

32. Un procédé selon la revendication 30 selon laquelle la composition pharmaceutique préparée est à utiliser comme un agent antinéoplastique.

33. Un procédé selon la revendication 30 selon laquelle la composition pharmaceutique préparée est à utiliser comme un agent antiviral.
